# EUROPEAN PATENT APPLICATION

(11) **EP 4 541 186 A2**
(43) Date of publication of application: **23.04.2025**
(21) Application number: 25154107.4
(22) Date of filing: 20.01.2020
(51) Int. Cl.: A01N 1/00

(54) **FUNCTIONAL WATER**

(30) Priority: 28.01.2019 JP 2019012627; 07.02.2019 JP 2019021024
(62) Divisional of application: 20747979.1
(71) Applicant: Kubota, Toru, Kobe-shi, Hyogo, 654-0151 (JP)
(72) Inventor: Kubota, Toru, Kobe-shi, Hyogo, 654-0151 (JP)
(74) Representative: Grünecker Patent- und Rechtsanwälte PartG mbB

(57) **Abstract**

A functional water containing a polyfunctional amine and/or a salt thereof as a functional component, wherein the total content of water, the polyfunctional amine, and the salt of the polyfunctional amine is 95% by weight or more. Desirably, the polyfunctional amine is at least one compound selected from the group consisting of a specific polyamine, a polymer having a structural unit derived from a specific cyclic amine, and a polymer having a structural unit derived from a specific unsaturated amine. The total content of the polyfunctional amine and/or salt thereof is preferably 0.0001 to 10000 ppm by weight. The functional water has functions including freshness-keeping for foods, aging of foods, antisepsis, deodorization, adjustment of plant growth, life extension for cut flowers, flowering control for cut flowers, pest control, pest repellence, improvement of bowel movement, reduction of fecal odor, reduction of blood pressure, increase of body temperature, improvement of the intraoral environment, prevention of halitosis, and prevention of body odor.

## Description

### Technical Field

The present invention relates to a novel functional water having a wide variety of functions.

### Background Art

Waters said to have a wide variety of functions such as sterilization, cleansing, deodorization, health promotion, rust prevention, and promotion of plant growth, have been previously introduced into the market.

For example, Patent Literature 1 describes: an electrolyzer and electrolysis apparatus with improved oxidation resistance imparted to an anion exchange membrane; and a method for producing an acidic electrolyzed water and a method for producing an alkaline electrolyzed water with use of an electrolysis apparatus including the electrolyzer. According to the description, the acidic electrolyzed water is used for sterilization, virucidal application, and deodorization, and the alkaline electrolyzed water is used for deodorization, degreasing, and removal of microparticles.

Patent Literature 2 describes a gold-ultramicroparticle-containing drinking water with gold ultramicroparticles suspended and dispersed in water. According to the description, the gold-ultramicroparticle-containing functional water of Patent Literature 2 can be directly drunk as a health drinking water, and the functional water is applicable as a main component or accessory component of health-promoting agents, cosmetics, antiseptics for foods, freshness-keeping agents for foods, insect repellents, deodorants, and so on.

Patent Literature 3 describes a rust-preventing functional water being slightly acidic or slightly alkaline, wherein the dissolved hydrogen/dissolved oxygen (molarity ratio) is at least 1. According to the description, the rust-preventing functional water has a function of preventing rusting in inner periphery surfaces of water supply pipes, and can be preferably used for beverages and other domestic water or daily life water.

Patent Literature 4 describes a method for cultivating a plant, wherein a plant is cultivated with a cultivating water containing hydrogen converted into protium. According to the description, the cultivating water enables promotion of plant growth, life extension for plants, and control of flowering and so on.

### Citation List

### Patent Literature

Patent Literature 1: Japanese Patent Laid-Open No. 2010-133007
Patent Literature 2: Japanese Patent Laid-Open No. 2008-214755
Patent Literature 3: Japanese Patent Laid-Open No. 2005-224696
Patent Literature 4: Japanese Patent Laid-Open No. 2012-55225

### Summary of Invention

### Technical Problem

The waters described in Patent Literatures 1, 3, and 4 are said to be effective only for specific uses. Thus, the application is restricted. The functional component in Patent Literature 2, gold, is a noble metal, and an expensive material. Accordingly, the water of Patent Literature 2 is poor in versatility, and continuous use of it is disadvantageous also in terms of cost. The waters described in Patent Literatures 1 to 3 are each produced by using a special production apparatus, and hence capital investment is inevitably needed. In order to use the cultivating water described in Patent Literature 4, it is essential to obtain a special magnetic ceramic ball, which is said to have strong reducibility.

Because of the above-described problems, the waters described in Patent Literatures 1 to 4 are by no means readily available to everyone, and all of them are not widely used in the world still now.

Thus, an object of the present invention is to provide a functional water that can be prepared in a simple manner and has an excellent function.

Another object of the present invention is to provide a functional water that can be prepared in a simple manner and has a wide variety of functions.

Another object of the present invention is to provide a material for preparing the functional water.

Another object of the present invention is to provide a health drinking water that can be prepared in a simple manner and exhibits an excellent function.

Another object of the present invention is to provide a bottled water, including the health drinking water packed in a container.

Another object of the present invention is to provide a health-promoting agent that exhibits an excellent function.

### Solution to Problem

The present inventor soaked a fresh cabbage in a water containing a certain polyfunctional amine and stored it at normal temperature for several years, and found by chance that the decomposition of the cabbage had not progressed at all and the state at the beginning of soaking was maintained. Further study on the water found that the water has a wide variety of functions such as high safety that allows drinking without any stimulation to the skin, and sterilization ability (antimicrobial function). In addition, further extensive study found that a novel functional water having a wide variety of functions can be obtained in a simple manner by blending a specific amount of a polyfunctional amine and/or a salt thereof as a functional component with water. The present invention was completed through additional examinations based on those findings.

Specifically, the present invention provides a functional water containing a polyfunctional amine and/or a salt thereof as a functional component, wherein the total content of water, the polyfunctional amine, and the salt of the polyfunctional amine is 95% by weight or more.

It is preferable that the polyfunctional amine in the functional water be at least one compound selected from the group consisting of a polyamine represented by formula (1): 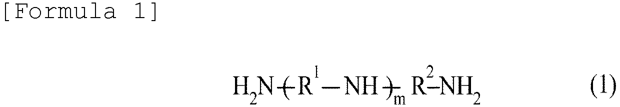
wherein m represents an integer of 0 to 1000, R¹ and R² each independently represent a linear or branched alkylene group having two to eight carbon atoms, and if m is 2 or more, a plurality of R¹ are the same or different;
a polymer having a structural unit derived from a cyclic amine represented by formula (2): 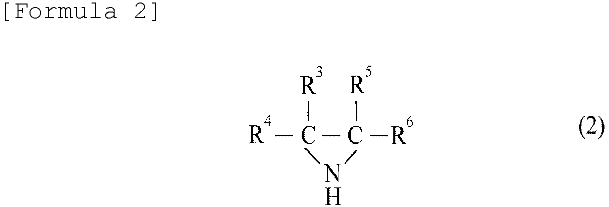
wherein R³, R⁴, R⁵, and R⁶ each independently represent a hydrogen atom or a methyl group; and
a polymer having a structural unit derived from an unsaturated amine represented by formula (3): 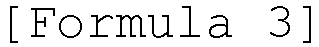 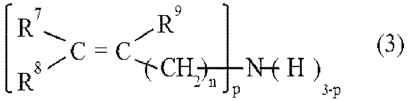
wherein n represents an integer of 0 to 2, p represents an integer of 1 to 3, and R⁷, R⁸, and R⁹ each independently represent a hydrogen atom or a methyl group.

It is preferable that the total content of the polyfunctional amine and/or salt thereof in the functional water be 0.0001 to 10000 ppm by weight.

It is preferable that the functional water have at least one function of freshness-keeping for foods, aging of foods, antisepsis, deodorization, cleansing, rust prevention, adjustment of plant growth, life extension for cut flowers, flowering control for cut flowers, pest control, pest repellence, parasite prevention, antimicrobial function, antiviral function, improvement of bowel movement, reduction of fecal odor, reduction of blood pressure, increase of body temperature, reduction of urinary glucose, reduction of blood glucose, anticancer function, mitigation of side effects of anticancer agents, antidepressant function, antischizophrenic function, anti-heart-disease function, antiasthmatic function, antirheumatic function, anti-Parkinson function, antigout function, function against connective tissue disease, anti-Alzheimer function, promotion of hair blackening, anti-hair-graying function, improvement of the intraoral environment, prevention of halitosis, prevention of body odor, pain relief, promotion of healing of insect bites, promotion of wound healing, promotion of burn healing, amelioration of peripheral nerve disorder, and antiinflammatory function. It is more preferable that the functional water be a functional water having, for example, two or more of the functions.

In addition, the present invention provides a material for preparing the functional water, the material containing a polyfunctional amine and/or a salt thereof.

It is preferable that the total content of the polyfunctional amine and/or salt thereof in the material be more than 0.0001 ppm by weight and not more than 100% by weight based on the total amount of the material.

In addition, the present invention provides a health drinking water containing a polyfunctional amine and/or a salt thereof as an active ingredient, wherein
the polyfunctional amine is at least one compound selected from the group consisting of a polyamine represented by formula (1): 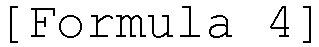 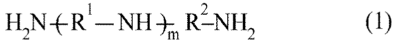
wherein m represents an integer of 0 to 1000, R¹ and R² each independently represent a linear or branched alkylene group having two to eight carbon atoms, and if m is 2 or more, a plurality of R¹ are the same or different;
a polymer having a structural unit derived from a cyclic amine represented by formula (2): 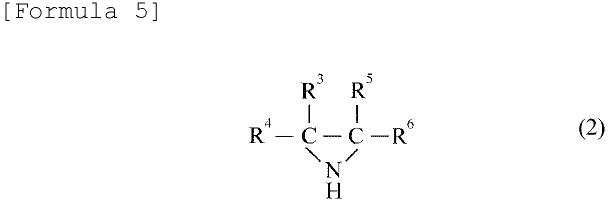
wherein R³, R⁴, R⁵, and R⁶ each independently represent a hydrogen atom or a methyl group; and
a polymer having a structural unit derived from an unsaturated amine represented by formula (3): 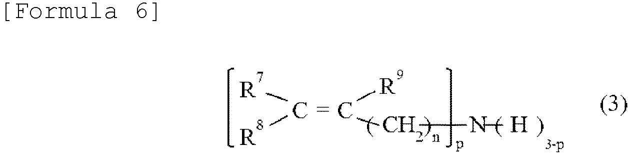
wherein n represents an integer of 0 to 2, p represents an integer of 1 to 3, and R⁷, R⁸, and R⁹ each independently represent a hydrogen atom or a methyl group.

It is preferable that the total content of the polyfunctional amine and/or salt thereof in the health drinking water be 0.0001 to 10000 ppm by weight.

In addition, the present invention provides a bottled water, including the health drinking water packed in a container.

Further, the present invention provides a material for preparing the health drinking water, the material containing a polyfunctional amine and/or a salt thereof.

The total content of the polyfunctional amine and/or salt thereof in the material for preparing the health drinking water may be more than 0.0001 ppm by weight and not more than 100% by weight based on the total amount of the material.

In addition, the present invention provides a health-promoting agent containing a polyfunctional amine and/or a salt thereof as an active ingredient, wherein
the polyfunctional amine is at least one compound selected from the group consisting of a polyamine represented by formula (1): 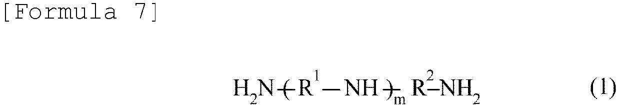
wherein m represents an integer of 0 to 1000, R¹ and R² each independently represent a linear or branched alkylene group having two to eight carbon atoms, and if m is 2 or more, a plurality of R¹ are the same or different;
a polymer having a structural unit derived from a cyclic amine represented by formula (2): 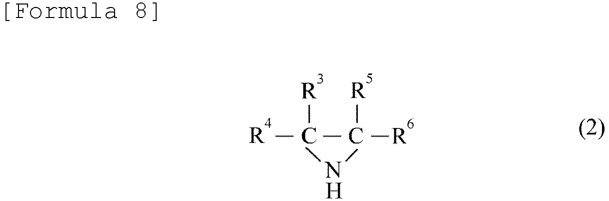
wherein R³, R⁴, R⁵, and R⁶ each independently represent a hydrogen atom or a methyl group; and
a polymer having a structural unit derived from an unsaturated amine represented by formula (3): 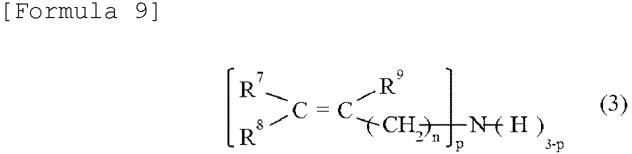
wherein n represents an integer of 0 to 2, p represents an integer of 1 to 3, and R⁷, R⁸, and R⁹ each independently represent a hydrogen atom or a methyl group.

It is preferable that the total content of the polyfunctional amine and/or salt thereof in the health-promoting agent be 0.0001 to 10000 ppm by weight.

### Advantageous Effects of Invention

The present invention provides a novel functional water and health drinking water that can be prepared in a simple manner and have excellent functions. The functional water of the present invention contains a polyfunctional amine and/or a salt thereof as a functional component or active ingredient, and can exhibit various functions by setting the total content of water, the polyfunctional amine, and the salt of the polyfunctional amine to 95% by weight or more. The health drinking water of the present invention contains a specific polyfunctional amine and/or salt thereof as an active ingredient, and thereby can exhibit various health-promoting actions.

Examples of the functions can include freshness-keeping for foods, aging of foods, antisepsis, deodorization, cleansing, rust prevention, adjustment of plant growth, life extension for cut flowers, flowering control for cut flowers, pest control, pest repellence, antimicrobial function, antiviral function, improvement of bowel movement, reduction of fecal odor, reduction of blood pressure, increase of body temperature, reduction of urinary glucose, reduction of blood glucose, anticancer function, mitigation of side effects of anticancer agents, antidepressant function, antischizophrenic function, anti-heart-disease function, antiasthmatic function, antirheumatic function, anti-Parkinson function, antigout function, function against connective tissue disease, anti-Alzheimer function, promotion of hair blackening, anti-hair-graying function, improvement of the intraoral environment, prevention of halitosis, prevention of body odor, pain relief, promotion of healing of insect bites, promotion of wound healing, promotion of burn healing, amelioration of peripheral nerve disorder, and antiinflammatory function. Hereinafter, the effects of the functions will be described in detail.

### [Antimicrobial Function, Antiviral Function, Cleansing Function]

The functional water of the present invention can have antimicrobial function and antiviral function. The functional water of the present invention is generally found to have efficacy to Escherichia coli, Staphylococcus aureus, and feline calicivirus, but not to have effect to Aspergillus niger. Although the accurate mechanism is not clear, the functional water of the present invention is inferred to have a certain selective antimicrobial function. Aspergillus niger is a mold that is used even in production of various types of enzyme agents and thus useful in the field of foods, and hence it is suggested that the functional water of the present invention is preferable particularly for hygiene management of working environments in the field of foods that handles Aspergillus niger. In contrast to sodium hypochlorite, the functional water of the present invention has neither corrosivity nor irritancy to the skin, and moreover has high safety to a degree that allows drinking. If the functional water of the present invention is used for washing fresh foods such as vegetables and fruits, in contrast to washing with a chlorine-containing agent, rinsing with a large amount of water is not needed. Hence, flavors inherent in foods are less likely to be deteriorated. If the functional water of the present invention is used for washing any vegetable for raw consumption such as cut vegetables (especially, welsh onion, green shiso, and Japanese ginger, which are used for flavoring), for example, the fragrance is not lost, and thus the product value is not decreased. In contrast to conventional agents, the functional water of the present invention does not need to be washed away, in general. For this reason, usage of water in food production processes can be reduced, which also leads to reduction of production cost.

### [Rust-Preventing Function]

The functional water of the present invention can have excellent rest-preventing function. Accordingly, the functional water of the present invention significantly reduces time and cost for maintenance of metallic equipment for food production or in food processing factories, kitchen instruments, cooking utensils, and so on. If the functional water of the present invention has the above-described antimicrobial function, antiviral function, and cleansing function in combination, equipment for food production or in food processing factories and working environments of kitchens are always kept sanitary, automatically through daily use of the functional water of the present invention as water for food production. Accordingly, it is not needed to purchase or use different agents such as rust-preventing agents and microbicides for different applications. In addition, high safety for workers and low environmental loads are simultaneously provided.

### [Freshness-Keeping Function for Foods, Aging Function for Foods, Antiseptic Function, Parasite-Preventing Function]

The functional water of the present invention can exhibit freshness-keeping function for foods through the above-described antimicrobial function. Accordingly, the functional water of the present invention can keep freshness without causing decomposition of foods, in general. For example, a live fish or a slice of fish is soaked in the functional water of the present invention and then vacuum-sealed, and this can be packed in a cardboard box and distributed to the market at normal temperature or under cooling. Conventional methods employed to distribute fish and shellfish with the freshness kept are conveying fish on a live fish car, which includes a water tank (container) filled with seawater for conveying live fish, while fish kept living are allowed to swim, and conveying on a freezer car or refrigerator car storing fish and shellfish in a container filled with ice. However, the conventional conveyance methods require high conveyance cost and suffer from large loss of space. For conveyance on a live fish car or a freezer or refrigerator car, it is needed to prepare new seawater or ice on each conveyance and to maintain and manage an expensive live fish car or the like. Moreover, huge cost is required, for example, for disposal of polluted seawater or ice and used containers after conveyance. In contrast to this, the functional water of the present invention avoids the need of conveyance on a special live fish car or the like or a container filled with a large amount of ice, as well as the need of disposal cost for polluted seawater or ice, and used containers. Thus, the transportation cost can be significantly reduced. Further, the functional water of the present invention can have function of aging foods to increase umami components. Use of the functional water of the present invention simultaneously as a freshness-keeping solution, as an antiseptic solution, and as an aging solution, for example, can provide not only reduced distribution cost, but also the expectation that deep taste due to aging will be generated, which is an unexpected synergistic effect. For this reason, foods treated with the functional water of the present invention have enhanced added values as products. In addition, the functional water of the present invention provides superior working efficiency because multiple steps including freshness-keeping, antisepsis, and aging for foods can be performed in one simple operation. Furthermore, the functional water of the present invention can exert parasite (Anisakis)-preventing function. While conventional methods employed to prevent anisakiasis are, for example, heating treatment (e.g., at 60°C for 1 minute) and freezing treatment (e.g., freezing at -20°C or less for 24 hours or more), the treatment methods deteriorate taste if being used for fresh fish and shellfish for raw consumption, and thus are not preferred. In contrast to this, the functional water of the present invention exhibits parasite-preventing function only by soaking fresh fish and shellfish therein, and hence enables prevention of anisakiasis without deteriorating the taste. Furthermore, the functional water of the present invention can have the above-described functions including freshness-keeping, aging, and antisepsis in combination, and hence is extremely useful as treatment water for fish and shellfish for raw consumption (water for food production).

### [Plant-Growth-Adjusting Function]

The functional water of the present invention can have plant-growth-adjusting function. In general, the functional water of the present invention exhibits function of promoting plant growth in a specific concentration range and suppressing plant growth in another specific concentration range. The functional water of the present invention can be expected to provide effects of improving yields and quality, enhancing disease resistance, and so on, for plants (crops). If the functional water of the present invention has the above-described antimicrobial effect in combination in using for hydroponic cultivation, retention of water quality of cultivating water is expected to be additionally achieved, and thus the time for maintenance is significantly reduced. This very probably leads to further improvement of production efficiency.

### [Life Extension for Cut Flowers, Flowering-Adjusting Function for Cut Flowers]

The functional water of the present invention can have life-extending function for cut flowers and flowering-adjusting function for cut flowers. In the current flower industry, various efforts to further improve freshness and longevity of flowers have been made, such as customization of distribution systems **(e.g.,** storage in temperature management facilities, transportation on refrigerator trucks or the like) and use of many types of agents including various antibacterial agents and preservatives. However, all of these involve significantly increased cost. In contrast to this, the functional water of the present invention enables life extension and flowering adjustment for cut flowers in an unexpensive and simple manner without need of special temperature management. Hence, the functional water of the present invention is very useful in any stage of producing areas, markets, retail stores, and consumers. In addition, the functional water of the present invention enables significant reduction of storage/transportation cost for cut flowers. Moreover, the functional water of the present invention has function of promoting development/opening of buds of lilies and so on, which have been harvested in the bud phase. Thus, flowering dates can be arbitrarily adjusted by controlling speed of development of flower buds and that of petal opening. Ultimately, flowering can be controlled so that flowering can occur all at once on a day with demands thereof, such as a day of a specific event or ceremony, obon, higan, and the New Year. Modulation of flowering in this manner can reduce loss to the minimum, and eventually increase profit rates for producers. Herein, "cut flowers" refer to a product obtained by cutting a plant with a flower or bud at an appropriate part primarily for conveyance or storage, or a product obtained by appropriately pruning a stem, a branch, a leaf, a flower, and a bud primarily for ornamental uses.

### [Pest Control, Pest-Controlling Function]

The functional water of the present invention can have control function against pests. When being sprinkled, the functional water of the present invention, in which water clusters are small, is inferred to exert the control function by clogging spiracles of pests such as mites, aphids, and scale insects to block air, causing suffocation. Conventionally, pyrethroid insecticides, organophosphate insecticides, carbamate insecticides, various synthetic miticides, and so on have been used for pest control. However, these agents are not necessarily satisfactory in terms of safety for humans. In contrast to this, the functional water of the present invention can exert pest-controlling function without causing generation of tolerance even when being prepared for use to a degree that allows drinking. If the functional water of the present invention is sprinkled on tree bark, the polyfunctional amine left on the tree bark is expected to exert repellent effect to prevent pests from laying eggs on the tree bark.

### [Deodorizing Function]

The functional water of the present invention can exhibit excellent deodorizing function to any of ammonia, hydrogen sulfide, and trimethylamine, which are malodorous substances. Malodors such as the odor of tobacco, the odor of Korean barbecues, the odor of elderly care, and the odor of pets can be erased by spraying or sprinkling the functional water of the present invention packed in a spray container or the like in the air or in a structure such as a building. Even tar stains attached on walls can be removed, for example, by one wipe with use of the functional water of the present invention. If the functional water of the present invention additionally has the above-described antimicrobial function, antiviral function, cleansing function, and so on in combination, the synergistic effect of them is probably obtained. The functional water of the present invention can be preferably used, for example, in the clinical environment, the care environment, the food industry, the food service industry, or amusement facilities.

[Improvement of Bowel Movement, Reduction of Fecal Odor, Reduction of Blood Pressure, Increase of Body Temperature, Reduction of Urinary Glucose, Reduction of Blood Glucose, Anticancer Function, Mitigation of Side Effects of Anticancer Agents, Antidepressant Function, Antischizophrenic Function, Anti-Heart-Disease Function, Antiasthmatic Function, Antirheumatic Function, Anti-Parkinson Function, Antigout Function, Function Against Connective Tissue Disease, Anti-Alzheimer Function, Promotion of Hair Blackening, Anti-Hair-Graying Function, Improvement of Intraoral Environment, Prevention of Halitosis, Prevention of Body Odor, Pain Relief, Promotion of Healing of Insect Bites, Promotion of Wound Healing, Promotion of Burn Healing, Amelioration of Peripheral Nerve Disorder, and Antiinflammatory Function, Etc.]

The functional water of the present invention can have the listed functions beneficial for health of humans and other animals (e.g., pets, domestic animals). Accordingly, if being ingested on a daily basis, the functional water of the present invention largely contributes to improvement or maintenance of a health state, thus to extension of healthy life expectancy. Although the accurate mechanism is not clear, the above-described selective antimicrobial function possessed by the functional water of the present invention is inferred to contribute to improvement of the balance of intestinal condition in humans and so on. This is expected to improve bowel movement and reduce fecal odor, leading to maintenance of health state. In addition, a phenomenon that gray hair gradually changes to black hair can occur through continuous drinking of the functional water of the present invention. Moreover, continuous drinking of the functional water of the present invention can cause to a person a phenomenon that the person is provided with enhanced concentration, high motivation, and enhanced stress tolerance, and hence antidepressant function is expected to be provided through daily ingestion.

The health drinking water of the present invention has the same functions as the functional water of the present invention. Daily drinking of the health drinking water of the present invention can largely contribute to health promotion in everyday life.

The health drinking water of the present invention can be packed in a container to use as a bottled water (hereinafter, occasionally referred to as "the bottled water of the present invention"). The usability is further improved by the form of a bottled water, which is very advantageous for portable use, transportation, storage, and distribution. If the functional water of the present invention has the above-described wide variety of functions in combination, the functions can be utilized in different manners in different scenes only with one bottled water. For example, it is contemplated that the functional water of the present invention is ingested as a soft drink for health promotion in a situation, packed in a spray container and sprayed as a deodorant in another situation, and sprayed to a food to keep the freshness of the food in still another situation. Thus, with the functional water of the present invention alone, the functions thereof can be utilized in different manners for different purposes, and hence the functional water of the present invention is very useful.

The present invention provides, in another aspect, a material for preparing a functional water (hereinafter, occasionally referred to as "the material of the present invention for preparing a functional water"), and a material for preparing a health drinking water (hereinafter, occasionally referred to as "the material of the present invention for preparing a health drinking water"). By diluting the material of the present invention for preparing a functional water and the material of the present invention for preparing a health drinking water, for example, with water, a required amount of the functional water and health drinking water of the present invention can be prepared at time of use in a simple manner. This enables effective use of storage space or the like without excessive loss of space, as compared with the case that the functional water itself is stored or distributed.

The present invention provides, in another aspect, a health-promoting agent (hereinafter, occasionally referred to as "the health-promoting agent of the present invention"). Through ingestion of the health-promoting agent of the present invention, various functions beneficial for health of humans and other animals (e.g.**,** pets, domestic animals) (improvement of bowel movement, reduction of fecal odor, reduction of blood pressure, increase of body temperature, reduction of urinary glucose, reduction of blood glucose, anticancer function, mitigation of side effects of anticancer agents, antidepressant function, antischizophrenic function, anti-heart-disease function, antiasthmatic function, antirheumatic function, anti-Parkinson function, antigout function, function against connective tissue disease, anti-Alzheimer function, promotion of hair blackening, anti-hair-graying function, improvement of the intraoral environment, prevention of halitosis, prevention of body odor, pain relief, promotion of healing of insect bites, promotion of wound healing, promotion of burn healing, amelioration of peripheral nerve disorder, antiinflammatory function, and so on) are expected to be exerted. Thus, the health-promoting agent of the present invention can be utilized for health promotion through daily ingestion.

### Brief Description of Drawings

[Figure 1] Figure 1 shows photographs demonstrating life-extending function 1 for cut flowers (rose) in Test Example 10.
[Figure 2] Figure 2 shows photographs demonstrating life-extending function 2 for cut flowers (Texas bluebell: Part 1) in Test Example 11.
[Figure 3] Figure 3 shows photographs demonstrating life-extending function 3 for cut flowers (Texas bluebell: Part 2) in Test Example 12.
[Figure 4] Figure 4 shows photographs demonstrating flowering-adjusting function 1 for cut flowers (Texas bluebell) in Test Example 13.
[Figure 5] Figure 5 shows photographs demonstrating flowering-adjusting function 2 for cut flowers (lily) in Test Example 14.
[Figure 6] Figure 6 shows photographs demonstrating flowering-adjusting function 3 for cut flowers (rose) in Test Example 15.
[Figure 7] Figure 7 shows photographs demonstrating plant-growth-adjusting function 1 (pea sprouts) in Test Example 16 for Comparative Example 4 and Example 1.
[Figure 8] Figure 8 shows photographs demonstrating plant-growth-adjusting function 1 (pea sprouts) in Test Example 16 for Examples 4 to 8.
[Figure 9] Figure 9 shows a photograph demonstrating Test Example plant-growth-adjusting function 2 (sprouting of soybeans) in Test Example 17.
[Figure 10] Figure 10 shows a photograph demonstrating plant-growth-adjusting function 3 (Persian buttercup) in Test Example 18.
[Figure 11] Figure 11 shows photographs demonstrating antiinflammatory function 1 day after application of Example 1 to an affected part of a sprained left big toe in Test Example 30.
[Figure 12] Figure 12 shows photographs demonstrating life-extending function 4 for cut flowers (carnation) in Test Example 47.

### Description of Embodiments

### [Functional Water]

The functional water of the present invention contains a polyfunctional amine and/or a salt thereof as a functional component, wherein the total content of water, the polyfunctional amine, and the salt of the polyfunctional amine is 95% by weight or more. Herein, a "functional water" refers to a water having a useful function that common water (e.g., pure water) does not originally have, and is not a registered trademark.

The functional water of the present invention may contain a component other than water and a polyfunctional amine and/or a salt thereof. For each of the components contained in the functional water of the present invention, for example, a polyfunctional amine and/or a salt thereof, only one substance or two or more substances may be used.

### (Water)

Examples of the water used for the functional water of the present invention can include natural water, tap water, ion-exchanged water, deionized water, distilled water, and purified water.

The content of water in the functional water of the present invention is, for example, 80% by weight or more, preferably 85% by weight or more, more preferably 90% by weight or more, and even more preferably 95% by weight or more (e.g., 98% by weight or more, 99% by weight or more, 99.5% by weight or more, 99.95% by weight or more, 99.98% by weight or more), to 100% by weight of the functional water of the present invention.

### (Polyfunctional Amine and/or Salt Thereof)

The polyfunctional amine used for the functional water of the present invention is a compound having two or more amino groups in one molecule, the scope of which includes polymers.

Each amino group possessed by the polyfunctional amine may be an amino group constituting any of primary amine, secondary amine, and tertiary amine.

### <Polyamine>

Examples of the polyfunctional amine include a polyamine represented by formula (1): 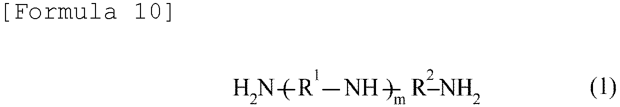 wherein m represents an integer of 0 to 1000, R¹ and R² each independently represent a linear or branched alkylene group having two to eight carbon atoms, and if m is 2 or more, a plurality of R¹ are the same or different.

R¹ and R² each preferably independently represent a linear or branched alkylene group having two to six carbon atoms, and even more preferably a linear or branched alkylene group having three to five carbon atoms.

m preferably represents an integer of 0 to 500, more preferably an integer of 0 to 100, even more preferably an integer of 0 to 50, and particularly preferably an integer of 0 to 10 (e.g., 0 to 5).

Examples of polyamines with m being 0 among polyamines represented by formula (1) include ethylenediamine, 1,2-diaminopropane, 1,3-diaminopropane, 1,4-diaminobutane (Putrescine), 1,5-diaminopentane, 1,6-diaminohexane, 1,7-diaminoheptane, and 1,8-diaminooctane.

Examples of polyamines with m being 1 or more among polyamines represented by formula (1) include spermidine, spermine; polyethyleneamine such as diethylenetriamine, triethylenetetramine, tetraethylenepentamine, pentaethylenehexamine, hexaethyleneheptamine, heptaethyleneoctamine, octaethylenenonamine, and nonaethylenedecamine; and polypropylenepolyamine such as dipropylenetriamine, tripropylenetetramine, tetrapropylenepentamine, pentapropylenehexamine, hexapropyleneheptamine, heptapropyleneoctamine, octapropylenenonamine, and nonapropyldecamine.

Commercially available products can be used as a polyamine represented by formula (1). Table 1 shows examples of such commercially available products.

### [Table 1]

**(Table 1)**

| Component name | Product name | Molecular weight | Manufacturer |
|---|---|---|---|
| Pentamethylenediamine | Pentamethylenediamine | 102.18 | Tokyo Chemical Industry Co., Ltd |
| Hexamethylenediamine | Hexamethylenediamine | 116.21 | Tokyo Chemical Industry Co., Ltd |
| Putrescine | Putrescine | 88.15 | MP Biomedicals,Inc |
| Spermidine | Spermidine, for molecular biology | 145.25 | FUJIFILM Wako Pure Chemical Corporation |
| 1,3-Propanediamine | 1,3-Propanediamine, Wako 1st Grade | 74.12 | FUJIFILM Wako Pure Chemical Corporation |

Among polyamines represented by formula (1), those such that m represents an integer of 0 to 10 (in particular, an integer of 0 to 5), and R¹ and R² each independently represent a linear or branched alkylene group having three to six carbon atoms are preferred.

### <Polymer Having Structural Unit Derived from Cyclic Amine>

An example of the above polyfunctional amine is a polymer having a structural unit derived from a cyclic amine represented by formula (2) (hereinafter, occasionally referred to as "polymer A"). Polymer A is obtained through ring-opening polymerization of a cyclic amine represented by formula (2): 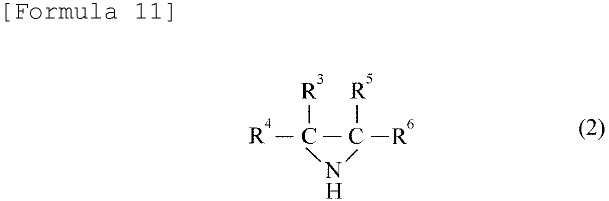 wherein R³, R⁴, R⁵, and R⁶ each independently represent a hydrogen atom or a methyl group.

Examples of cyclic amines represented by formula (2) include aziridine (ethyleneimine), 2-methylaziridine, 2,2-dimethylaziridine, and 2,3-dimethylaziridine. Examples of homopolymers as polymer A include polyethyleneimine, poly(2-methylaziridine), poly(2,2-dimethylaziridine), and poly(2,3-dimethylaziridine).

Commercially available products can be used as polymer A. Table 2 shows examples of such commercially available products.

### [Table 2]

**(Table 2)**

| Component name | Product name | Molecular weight | Manufacturer |
|---|---|---|---|
| Polyethyleneimine | Polyethyleneimine (approx. 30% aqueous solution) | 70,000 (weight-average molecular weight) | Tokyo Chemical Industry Co., Ltd |

### <Polymer Having Structural Unit Derived from Unsaturated Amine>

An example of the above polyfunctional amine is a polymer having a structural unit derived from an unsaturated amine represented by formula (3) (hereinafter, occasionally referred to as "polymer B"). Polymer B is obtained through polymerization of unsaturated groups of an unsaturated amine represented by formula (3): 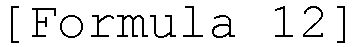 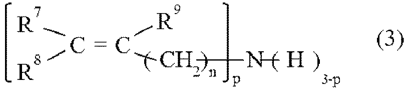 wherein n represents an integer of 0 to 2, p represents an integer of 1 to 3, and R⁷, R⁸, and R⁹ each independently represent a hydrogen atom or a methyl group.

Examples of unsaturated amines represented by formula (3) include vinylamine, divinylamine, trivinylamine, allylamine, diallylamine, triallylamine, methallylamine, dimethallylamine, trimethallylamine, crotylamine, dicrotylamine, tricrotylamine, 3-methyl-2-butenylamine, and 3-butenylamine.

Among unsaturated amines represented by formula (3), compounds such that n represents 0 or 1, p represents 1 or 2, and R⁷, R⁸, and R⁹ are each a hydrogen atom are preferred.

Examples of homopolymers as polymer B include polyvinylamine, polydivinylamine, polyallylamine, polydiallylamine, polytriallylamine, polymethacrylamine, polycrotylamine, and poly(3-butenylamine).

The weight-average molecular weight of polymer B is, for example, 100 to 200,000, preferably 300 to 100,000, and even more preferably 500 to 50,000.

Polymer A and polymer B may be each not only any of the above homopolymers but also a copolymer. Regarding the mode of arrangement of the copolymer, any of statistical copolymer, random copolymer, alternating copolymer, and periodic copolymer is acceptable, and regarding the manner of linkage of polymer chains, any of block copolymer and graft copolymer is acceptable.

In the case that polymer A is a copolymer, polymer A may be a copolymer of two or more cyclic amines represented by formula (2), and in the case that polymer B is a copolymer, polymer B may be a copolymer of two or more of unsaturated amines represented by formula (3) or a copolymer of one or two or more of unsaturated amines represented by formula (3) and any other monomer copolymerizable with these unsaturated amines represented by formula (3).

Examples of other monomers copolymerizable with unsaturated amines represented by formula (3) include sulfur dioxide, (meth)acrylamide, (meth)acrylate, (meth)acrylic acid, and maleic acid.

In the case that polymer B is a copolymer, the fraction of structural units derived from any of unsaturated amines represented by formula (3) (including those with a substituent introduced to an amino group and those forming a salt) to the entire copolymer is, for example, 20% by weight or more, preferably 40% by weight or more, more preferably 60% by weight or more, even more preferably 80% by weight or more, and particularly preferably 90% by weight or more.

In the case that polymer B is a copolymer, the fraction of structural units derived from any of unsaturated amines represented by formula (3) without a substituent introduced to an amino group (including those in which an amino group is forming a salt) to the entire copolymer is, for example, 20% by weight or more, preferably 40% by weight or more, more preferably 60% by weight or more, even more preferably 80% by weight or more, and particularly preferably 90% by weight or more.

In any of the polyamine represented by formula (1), polymer A, and polymer B, any amino group in the molecule may have a substituent or have no substituent.

Examples of the substituent include alkyl groups having one to four carbon atoms such as a methyl group and an ethyl group; acyl groups having one to four carbon atoms such as a formyl group, an acetyl group, and a propionyl group; alkoxycarbonyl groups in which the alkoxy moiety has one to four carbon atoms such as a methoxycarbonyl group and an ethoxycarbonyl group; and a carbamoyl group. Such a substituent may have been introduced to a monomer before polymerization or introduced after polymerization.

The polyfunctional amine used for the functional water of the present invention is preferably at least one compound selected from the group consisting of a polyamine represented by formula (1), polymer A, and polymer B. Especially for superior economic efficiency and availability, the polyfunctional amine used for the functional water of the present invention is more preferably any of polymer A and/or polymer B, and the most preferably polymer B.

Representative examples of polymers having a structural unit derived from an unsaturated amine represented by formula (3), that is, polymer B include homopolymers including polyvinylamine, polydivinylamine, polyallylamine, polydiallylamine, polytriallylamine, polymethallylamine, polycrotylamine, poly(3-butenylamine), diallylamine polymer, poly(N,N-dimethylallylamine), and poly(N-acetylallylamine); and copolymers including allylamine-diallylamine copolymer, allylamine-dimethylallylamine copolymer, partially methoxycarbonylated allylamine copolymer, partially ureaized allylamine polymer, diallylamine-sulfur dioxide copolymer, methyldiallylamine-sulfur dioxide copolymer, diallylamine-acrylamide copolymer, allylamine-maleic acid copolymer, diallylamine-maleic acid copolymer, methyldiallylamine-maleic acid copolymer, partially formylated allylamine polymer, partially acetylated allylamine polymer, partially propionylated allylamine polymer, allylamine-N,N-dimethylallylamine copolymer, partially formylated diallylamine polymer, partially acetylated diallylamine polymer, and partially propionylated diallylamine polymer.

Commercially available products can be used as polymer B. Table 3 shows examples of such commercially available products. Polyvinylamine can be produced, for example, by using a method described in the specification of Japanese Patent No. 3511626.

### [Table 3]

**(Table 3)**

| Component name | Product name | Molecular weight | Manufacturer |
|---|---|---|---|
| Polyallylamine | PAA-01 (15% aqueous solution) | 1,600 (weight-average molecular weight) | Nittobo Medical Co., Ltd. |
| Polyallylamine | PAA-15C (15% aqueous solution) | 15,000 (weight-average molecular weight) | Nittobo Medical Co., Ltd. |
| Diallylamine polymer | PAS-21 (15% aqueous solution) | 5,000 (weight-average molecular weight) | Nittobo Medical Co., Ltd. |
| Polyallylamine | PAA-25 (10% aqueous solution) | 25,000 (weight-average molecular weight) | Nittobo Medical Co., Ltd. |
| Allylamine-dimethylallylamine copolymer* | PAA-1112 (15% aqueous solution) | 1,000 (weight-average molecular weight) | Nittobo Medical Co., Ltd. |
| Allylamine hydrochloride-diallylamine hydrochloride copolymer* | PAA-D11+HCL (40% aqueous solution) | 100,000 (weight-average molecular-weight) | Nittobo Medical Co., Ltd. |

| | | | |
|---|---|---|---|
| *The copolymerization ratio (mole ratio) of the copolymer is 1:1. | | | |

It is preferable for polymer B that two or more different polymer chains be not crosslinked via the nitrogen atom of an amino group derived from the unsaturated amine.

The salt of the polyfunctional amine may be any of salts with an acid and quaternary ammonium salts. Examples of the acid include inorganic acids such as hydrochloric acid, sulfuric acid, nitric acid, phosphoric acid, and boric acid; and organic acids including monofunctional aliphatic carboxylic acids such as formic acid, acetic acid, propionic acid, butyric acid, isobutyric acid, pentanoic acid, hexanoic acid, octanoic acid, decanoic acid, and dodecanoic acid; divalent aliphatic carboxylic acids such as oxalic acid, malonic acid, succinic acid, glutaric acid, and adipic acid; hydroxycarboxylic acids such as glycolic acid, lactic acid, malic acid, and tartaric acid; and sulfonic acids such as methanesulfonic acid and p-toluenesulfonic acid.

The total content of the polyfunctional amine and/or salt thereof in the functional water of the present invention is, for example, 0.0001 to 10000 ppm by weight, more preferably 0.001 to 9000 ppm by weight, even more preferably 0.01 to 8000 ppm by weight, particularly preferably 0.1 to 7000 ppm by weight, the most preferably 0.5 to 6000 ppm by weight, especially preferably more than 0.8 ppm by weight(e.g., more than 1 ppm by weight, preferably more than 2 ppm by weight, more preferably more than 3 ppm by weight, even more preferably more than 4 ppm by weight, particularly preferably more than 5 ppm by weight) and not more than 5000 ppm by weight (e.g.**,** 4000 ppm by weight or less, preferably 3000 ppm by weight or less, more preferably 2000 ppm by weight or less, even more preferably 1500 ppm by weight or less, particularly preferably 1000 ppm by weight or less).

In the following, preferred total contents of the polyfunctional amine and/or salt thereof will be shown for each function.

### [Antimicrobial Function, Antiviral Function, Cleansing Function, Rust-Preventing Function, Freshness-Keeping Function for Foods, Aging Function for Foods, Antiseptic Function, Pest Control, Pest Repellence]

A preferred lower limit value is 1 ppm by weight, more preferably 5 ppm by weight, and even more preferably 7.5 ppm by weight, and a preferred upper limit value is 5000 ppm by weight, more preferably, 3000 ppm by weight, and even more preferably 2000 ppm by weight.

### [Plant-Growth-Adjusting Function]

The functional water of the present invention has function of suppressing growth at high concentration and promoting growth at low concentration. The concentration to exert the plant-growth-promoting function is, for example, 0.0001 to 750 ppm by weight, preferably 0.001 to 500 ppm by weight, more preferably 0.005 to 250 ppm by weight, and even more preferably 0.01 to 100 ppm by weight (e.g., 0.01 to 75 ppm by weight, preferably 0.01 to 50 ppm by weight, more preferably 0.001 to 25 ppm by weight, even more preferably 0.01 to 10 ppm by weight). The concentration to exert the growth-suppressing function is, for example, 750 to 10000 ppm by weight, though the concentration depends on the type of a plant of interest. When plant-growth-promoting function is exerted at extremely low concentration, the growth-suppressing function may be exerted, for example, even at 0.25 ppm by weight or more (preferably 0.3 ppm by weight, more preferably 0.5 ppm by weight or more, even more preferably 0.75 ppm by weight or more).

### [Life Extension for Cut Flowers, Flowering-Adjusting Function for Cut Flowers (Buds)]

The concentration to exert the life-extending effect is, for example, 0.0001 to 500 ppm by weight, preferably 0.001 to 400 ppm by weight, more preferably 0.005 to 300 ppm by weight, and even more preferably 0.01 to 250 ppm by weight. The concentration to exert the flowering-adjusting function for cut flowers (including buds) is, for example, 0.0001 to 200 ppm by weight, preferably 0.001 to 150 ppm by weight, more preferably 0.005 to 100 ppm by weight, and even more preferably 0.01 to 75 ppm by weight.

[Improvement of Bowel Movement, Reduction of Fecal Odor, Reduction of Blood Pressure, Increase of Body Temperature, Reduction of Urinary Glucose, Reduction of Blood Glucose, Anticancer Function, Mitigation of Side Effects of Anticancer Agents, Antidepressant Function, Antischizophrenic Function, Anti-Heart-Disease Function, Antiasthmatic Function, Antirheumatic Function, Anti-Parkinson Function, Antigout Function, Function Against Connective Tissue Disease, Anti-Alzheimer Function, Promotion of Hair Blackening, Anti-Hair-Graying Function, Improvement of Intraoral Environment, Prevention of Halitosis, Prevention of Body Odor, Pain Relief, Promotion of Healing of Insect Bites, Promotion of Wound Healing, Promotion of Burn Healing, Amelioration of Peripheral Nerve Disorder, and Antiinflammatory Function]

The standard daily dose of the polyfunctional amine and/or salt thereof is, for example, 0.01 mg to 1500 mg, preferably 0.1 mg to 1000 mg, more preferably 0.2 mg to 500 mg, and particularly preferably 0.5 mg to 100 mg. In the case that the functional water with the total content of the polyfunctional amine and/or salt thereof being 75 ppm by weight is to be drunk, for example, drinking an absolute dose of 30 mL/day corresponds to ingestion of 2.25 mg/day of the polyfunctional amine and/or salt thereof.

### [Additional Components]

The functional water of the present invention may contain various additives as additional components, unless any of the advantageous effects of the present invention is deteriorated. For example, one or two or more of medicinal components, water-soluble dietary fibers, vitamins, minerals, juices, flavors, thickeners, viscosifiers, antiseptics, sweeteners, surfactants, antioxidants, emulsifiers, coloring agents, preservatives, pH adjusters, seasonings, acidulants, quality stabilizers, and so on may be blended as such additional components.

In order to allow the functional water of the present invention to exert the functions due to the action of the polyfunctional amine and/or salt thereof to the fullest extent, it is preferable that the functional water of the present invention be substantially free of components other than water, the polyfunctional amine, and the salt of the polyfunctional amine. Specifically, the content of the additional components to 100% by weight of the functional water of the present invention is preferably less than 5% by weight, more preferably less than 3% by weight, even more preferably less than 1% by weight, and particularly preferably zero. If the functional water of the present invention is substantially free of the additional components (less than 5% by weight), the polyfunctional amine and/or salt thereof is not interfered by the additional components in the aqueous solution, and hence the effects of the polyfunctional amine and/or salt thereof are more effectively exerted.

The functional water of the present invention contains a polyfunctional amine and/or a salt thereof as a functional component, wherein the total content of water, the polyfunctional amine, and the salt of the polyfunctional amine is, for example, 95% by weight or more, preferably 98% by weight or more, 99% by weight or more, more preferably 99.5% by weight or more, even more preferably 99.95% by weight or more, particularly preferably 99.98% by weight or more, and the most preferably 100% by weight.

The pH of the functional water of the present invention is, for example, 5.0 to 9.0, preferably 5.8 to 8.6, even more preferably 6.5 to 7.8, and particularly preferably 7.0 to 7.5. With the pH being in the range of 5.0 to 9.0, the functional water of the present invention is easy to drink without extreme acidity or bitterness. Further, the pH being in the range of 5.8 to 8.6 meets the pH criterion in the water quality standards based on the specification of the Water Supply Act in Japan. Furthermore, the neutral or weakly alkaline pH in the range of 7.0 to 7.5 gives much milder mouth feel, and facilitates adsorption in the body because the pH is close to the pH value in the body (approximately 7.4).

### [Production of Functional Water]

The functional water of the present invention can be produced in accordance with a known production method for beverages or a known production method for solutions in the field of medicines, with addition of a step of including the polyfunctional amine and/or salt thereof as a functional component.

For example, the functional water of the present invention can be produced by using the following method.

### (Step of Including Polyfunctional Amine and/or Salt Thereof)

The step is not limited as long as a homogeneous functional water can be obtained in the step by adding the polyfunctional amine and/or salt thereof to raw material water (e.g., natural water, tap water, ion-exchanged water, deionized water, distilled water, or purified water) and mixing/stirring the resultant.

The material of the present invention for preparing a functional water, which is described later, may be used as the polyfunctional amine and/or salt thereof. If the material of the present invention for preparing a functional water is used, the functional water can be produced in a much simpler manner. In the case that the material of the present invention for preparing a functional water is a concentrate, for example, the concentrate can be diluted in advance with part of the raw material water at a desired dilution rate and added as the form of aqueous solution. Particularly for the case that the concentrate is diluted to a very low concentration, stepwise dilution (e.g., base 10-fold diluted solution is further 10-fold diluted) allows accurate concentration adjustment in a simple manner, and thus the case is preferred. For example, if 10 mL is taken from commercially available mineral water in a container of a 2 L-plastic bottle, and 10 mL of the material of the present invention for preparing a functional water (e.g., amount of polyfunctional amine and/or salt thereof: 75 ppm by weight) is added thereto, then the functional water of the present invention with the total content of the polyfunctional amine and/or salt thereof being 0.375 ppm by weight is obtained, simply. As necessary, an additional component (e.g., a pH adjuster, a flavor) may be added in the step.

In place of using the above-described method, for example, the raw material water may be passed through a porous body including the polyfunctional amine and/or salt thereof (e.g., a product obtained by allowing a porous body to support thereon the polyfunctional amine and/or salt thereof), thereby allowing the raw material water to continuously include the polyfunctional amine and/or salt thereof.

Examples of the material constituting the porous body can include mesoporous silica, silica gel, silica/alumina, ion exchange resin, diatomaceous earth, activated carbon, zeolite, Japanese acid clay, ceramics, cellulose, various types of resins, nonwoven fabrics, and woven fabrics.

The system to pass the raw material water through the porous body is not limited, and, for example, there exists (1) a method in which a porous body supporting thereon the polyfunctional amine and/or salt thereof is placed in a water storage tank and the raw material water is fed thereinto, and (2) a method in which the porous body is set like a filtration filter in a part of a water supply line and the raw material water is continuously passed therethrough. A specific example of (2) is an embodiment like a common water purifier cartridge.

### (Other Steps)

Examples of other steps include a heat sterilization step (e.g., a method of heating at 60°C for 10 minutes, at 85°C for 30 minutes, or at 120°C for 4 minutes), a cooling step, a filtration step, a packing step, a sealing step, a testing step, and a packaging step.

The above method for producing a functional water enables inclusion of a certain ratio of the polyfunctional amine and/or salt thereof in the raw material water, and thus the functional water can be provided, simply and quickly.

### [Material for Preparing Functional Water]

The material of the present invention for preparing a functional water at least contains the polyfunctional amine and/or salt thereof as a functional component, and can further contain water and an additional component as listed above.

The material of the present invention for preparing a functional water is a material with which the above functional water can be prepared, for example, by diluting with water. The material of the present invention for preparing a functional water can be in any of various liquid forms including solution, emulsion, and suspension, and such a liquid form can be solidified or semi-solidified, or encapsulated to form any of various forms including gel, capsules, powder, particles, and granules.

The total content of the polyfunctional amine and/or salt thereof is, for example, more than 0.0001 ppm by weight, 100% by weight based on the total amount of the material of the present invention for preparing a functional water. The lower limit is preferably 0.1 ppm by weight, more preferably 1 ppm by weight, even more preferably 100 ppm by weight, and particularly preferably 10000 ppm by weight (e.g., 50000 ppm by weight). The upper limit is preferably 95% by weight, more preferably 90% by weight, even more preferably 80% by weight, and particularly preferably 70% by weight.

If the material of the present invention for preparing a functional water contains a component other than the polyfunctional amine and/or salt thereof, the material of the present invention for preparing a functional water can be prepared in the same manner as any known production method for foods and beverages, with addition of a step of including the polyfunctional amine and/or salt thereof as a functional component.

### [Health Drinking Water]

The health drinking water of the present invention contains a polyfunctional amine and/or a salt thereof as an active ingredient, wherein the polyfunctional amine is at least one compound selected from the group consisting of a polyamine represented by formula (1), wherein m represents an integer of 0 to 1000, R¹ and R² each independently represent a linear or branched alkylene group having two to eight carbon atoms, and if m is 2 or more, a plurality of R¹ are the same or different; a polymer having a structural unit derived from a cyclic amine represented by formula (2), wherein R³, R⁴, R⁵, and R⁶ each independently represent a hydrogen atom or a methyl group; and
a polymer having a structural unit derived from an unsaturated amine represented by formula (3), wherein n represents an integer of 0 to 2, p represents an integer of 1 to 3, and R⁷, R⁸, and R⁹ each independently represent a hydrogen atom or a methyl group. It is preferable that the total content of water, the polyfunctional amine, and the salt of the polyfunctional amine in the health
drinking water of the present invention be 95% by weight or more.

Details of the polyamine represented by formula (1), the polymer having a structural unit derived from a cyclic amine represented by formula (2), and the polymer having a structural unit derived from an unsaturated amine represented by formula (3) are the same as those described in the section "Functional Water".

The content of water in the health drinking water of the present invention is, for example, 80% by weight or more, preferably 85% by weight or more, more preferably 90% by weight or more, and even more preferably 95% by weight or more (e.g., 98% by weight or more, 99% by weight or more, 99.5% by weight or more, 99.95% by weight or more, 99.98% by weight or more) to 100% by weight of the health drinking water of the present invention.

The total content of the polyfunctional amine and/or salt thereof in the health drinking water of the present invention is, for example, 0.0001 to 10000 ppm by weight, more preferably 0.001 to 9000 ppm by weight, even more preferably 0.01 to 8000 ppm by weight, particularly preferably 0.1 to 7000 ppm by weight, the most preferably 0.5 to 6000 ppm by weight, and especially preferably more than 0.8 ppm by weight (e.g., more than 1 ppm by weight, preferably more than 2 ppm by weight, more preferably more than 3 ppm by weight, even more preferably more than 4 ppm by weight, particularly preferably more than 5 ppm by weight) and not more than 5000 ppm by weight (e.g., 4000 ppm by weight or less, preferably 3000 ppm by weight or less, more preferably 2000 ppm by weight or less, even more preferably 1500 ppm by weight or less, particularly preferably 1000 ppm by weight or less).

If the health drinking water of the present invention is drunk, the standard daily dose of the polyfunctional amine and/or salt thereof is, for example, 0.01 mg to 1500 mg, preferably 0.1 mg to 1000 mg, more preferably 0.2 mg to 500 mg, and particularly preferably 0.5 mg to 100 mg. In the case that the functional water with the total content of the polyfunctional amine and/or salt thereof being 75 ppm by weight is to be drunk, for example, drinking 30 mL/day, as an absolute dose per day, corresponds to ingestion of 2.25 mg/day of the polyfunctional amine and/or salt thereof.

Through daily drinking of the health drinking water of the present invention, functions including improvement of bowel movement, reduction of fecal odor, reduction of blood pressure, increase of body temperature, reduction of urinary glucose, reduction of blood glucose, anticancer function, mitigation of side effects of anticancer agents, antidepressant function, antischizophrenic function, anti-heart-disease function, antiasthmatic function, antirheumatic function, anti-Parkinson function, antigout function, function against connective tissue disease, anti-Alzheimer function, promotion of hair blackening, anti-hair-graying function, improvement of the intraoral environment, prevention of halitosis, prevention of body odor, pain relief, promotion of healing of insect bites, promotion of wound healing, promotion of burn healing, amelioration of peripheral nerve disorder, and antiinflammatory function are exerted together in combination. Thus, the health drinking water of the present invention can contribute to health promotion for humans and so on. Examples of particularly superior health-promoting action exerted by the health drinking water of the present invention include improvement of bowel movement, reduction of fecal odor, reduction of blood pressure, increase of body temperature, improvement of the intraoral environment, prevention of halitosis, and prevention of body odor.

With controlling the total content of the polyfunctional amine and/or salt thereof in the health drinking water of the present invention, the health drinking water of the present invention can be used even as "tap water" meeting water quality standards or "water for food production" meeting codes and standards for water for food production. Water meeting the standards is said to be safe water that humans can drink without worry.

To prepare the health drinking water of the present invention so as to meet standards for tap water or water for food production, the concentrations of nitrate nitrogen and nitrite nitrogen (10 mg/L or less) and pH value (not less than 5.8 and not more than 8.6) can be used as indicators for the total content of the polyfunctional amine and/or salt thereof in the health drinking water of the present invention. In general, water such as natural water, tap water, ion-exchanged water, deionized water, distilled water, and purified water can be used to adjust the total content of the polyfunctional amine and/or salt thereof in the health drinking water of the present invention.

The health drinking water of the present invention can be produced according to the method for producing the functional water of the present invention.

### [Bottled Water]

The bottled water of the present invention refers to the above health drinking water packed in a container. For the packing container to be used for the bottled water of the present invention, containers of any material in any form can be used. Examples of such packing containers include aluminum cans, steel cans, bottles, plastic bottles, barrels, pouches, paper containers, flasks, beakers, various types of capsule-shaped containers, and various types of laminate containers, in which a metal foil, a plastic film, and so on are laminated.

The bottled water of the present invention can be produced in accordance with a known production method for beverages, for example, including a bottling step involving packing the above health drinking water in a bottle for drinking water such as a can, a bottle, and a plastic bottle, and, as necessary, pasting on the bottle a label or the like with needed information written thereon.

### [Material for Preparing Health Drinking Water]

The material of the present invention for preparing a health drinking water at least contains the polyfunctional amine and/or salt thereof, and can further contain water and, as necessary, an additional component as listed above.

The material of the present invention for preparing a health drinking water is a material with which the above health drinking water can be prepared, for example, by diluting with water. The material of the present invention for preparing a health drinking water can be in any of various liquid forms including solution, emulsion, and suspension, and such a liquid form can be solidified or semi-solidified to form any of various forms including powder, particles, and granules.

The total content of the polyfunctional amine and/or salt thereof is, for example, more than 0.0001 ppm by weight and less than 100% by weight based on the total amount of the material of the present invention for preparing a health drinking water. The lower limit is preferably 0.1 ppm by weight, more preferably 1 ppm by weight, even more preferably 100 ppm by weight, and particularly preferably 10000 ppm by weight (e.g., 50000 ppm by weight). The upper limit is preferably 95% by weight, more preferably 90% by weight, even more preferably 80% by weight, and particularly preferably 70% by weight.

If the material of the present invention for preparing a health drinking water contains a component other than the polyfunctional amine and/or salt thereof, the material of the present invention for preparing a health drinking water can be prepared in the same manner as any known production method for beverages, with addition of a step of including the polyfunctional amine and/or salt thereof as a functional component.

### [Health-Promoting Agent]

The present invention provides, in another aspect, a health-promoting agent containing a polyfunctional amine and/or a salt thereof as an active ingredient, wherein
the polyfunctional amine is at least one compound selected from the group consisting of a polyamine represented by formula (1): 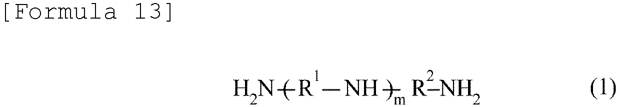
wherein m represents an integer of 0 to 1000, R¹ and R² each independently represent a linear or branched alkylene group having two to eight carbon atoms, and if m is 2 or more, a plurality of R¹ are the same or different;
a polymer having a structural unit derived from a cyclic amine represented by formula (2): 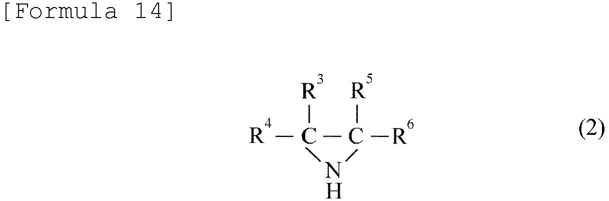
wherein R³, R⁴, R⁵, and R⁶ each independently represent a hydrogen atom or a methyl group; and
a polymer having a structural unit derived from an unsaturated amine represented by formula (3): 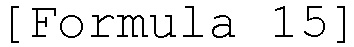 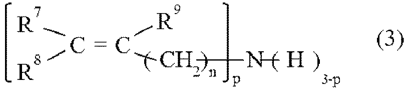
wherein n represents an integer of 0 to 2, p represents an integer of 1 to 3, and R⁷, R⁸, and R⁹ each independently represent a hydrogen atom or a methyl group.

Details of formulas (1) to (3) are the same as those described above in detail in the description of the functional water of the present invention.

Especially for lower cost and availability, the polyfunctional amine in the health-promoting agent of the present invention is more preferably any of the polymer having a structural unit derived from a cyclic amine represented by formula (2) and/or the polymer having a structural unit derived from an unsaturated amine represented by formula (3), and the most preferably the polymer having a structural unit derived from an unsaturated amine represented by formula (3).

In any of the polyamine represented by formula (1), the polymer having a structural unit derived from a cyclic amine represented by formula (2), and the polymer having a structural unit derived from an unsaturated amine represented by formula (3), any amino group in the molecule may have a substituent or have no substituent.

Examples of the substituent include alkyl groups having one to four carbon atoms such as a methyl group and an ethyl group; acyl groups having one to four carbon atoms such as a formyl group, an acetyl group, and a propionyl group; alkoxycarbonyl groups in which the alkoxy moiety has one to four carbon atoms such as a methoxycarbonyl group and an ethoxycarbonyl group; and a carbamoyl group. Such a substituent may have been introduced to a monomer before polymerization or introduced after polymerization.

The salt of the polyfunctional amine may be any of salts with an acid and quaternary ammonium salts. Examples of the acid include inorganic acids such as hydrochloric acid, sulfuric acid, nitric acid, phosphoric acid, and boric acid; and organic acids including monofunctional aliphatic carboxylic acids such as formic acid, acetic acid, propionic acid, butyric acid, isobutyric acid, pentanoic acid, hexanoic acid, octanoic acid, decanoic acid, and dodecanoic acid; divalent aliphatic carboxylic acids such as oxalic acid, malonic acid, succinic acid, glutaric acid, and adipic acid; hydroxycarboxylic acids such as glycolic acid, lactic acid, malic acid, and tartaric acid; and sulfonic acids such as methanesulfonic acid and p-toluenesulfonic acid.

The total content of the polyfunctional amine and/or salt thereof in the health-promoting agent of the present invention is, for example, 0.0001 to 10000 ppm by weight, more preferably 0.001 to 9000 ppm by weight, even more preferably 0.01 to 8000 ppm by weight, particularly preferably 0.1 to 7000 ppm by weight, the most preferably 0.5 to 6000 ppm by weight, and especially preferably more than 0.8 ppm by weight (e.g., more than 1 ppm by weight, preferably more than 2 ppm by weight, more preferably more than 3 ppm by weight, even more preferably more than 4 ppm by weight, particularly preferably more than 5 ppm by weight) and not more than 5000 ppm by weight (e.g., 4000 ppm by weight or less, preferably 3000 ppm by weight or less, more preferably 2000 ppm by weight or less, even more preferably 1500 ppm by weight or less, particularly preferably 1000 ppm by weight or less).

If the health-promoting agent of the present invention is drunk or taken, the standard daily dose of the polyfunctional amine and/or salt thereof is, for example, 0.01 mg to 1500 mg, preferably 0.1 mg to 1000 mg, more preferably 0.2 mg to 500 mg, and particularly preferably 0.5 mg to 100 mg. In the case that the health-promoting agent with the total content of the polyfunctional amine and/or salt thereof being 75 ppm by weight is to be drunk, for example, drinking an absolute dose of 30 mL/day corresponds to ingestion of 2.25 mg/day of the polyfunctional amine and/or salt thereof.

Through ingestion of the health-promoting agent of the present invention, various functions beneficial for health of humans and so on (e.g., improvement of bowel movement, reduction of fecal odor, reduction of blood pressure, increase of body temperature, reduction of urinary glucose, reduction of blood glucose, anticancer function, mitigation of side effects of anticancer agents, antidepressant function, antischizophrenic function, anti-heart-disease function, antiasthmatic function, antirheumatic function, anti-Parkinson function, antigout function, function against connective tissue disease, anti-Alzheimer function, promotion of hair blackening, anti-hair-graying function, improvement of the intraoral environment, prevention of halitosis, prevention of body odor, pain relief, promotion of healing of insect bites, promotion of wound healing, promotion of burn healing, amelioration of peripheral nerve disorder, and antiinflammatory function) are exerted. Thus, the health-promoting agent of the present invention can be utilized for health promotion in humans and so on through daily ingestion. Examples of particularly superior health-promoting action exerted by the health-promoting agent of the present invention include improvement of bowel movement, reduction of fecal odor, reduction of blood pressure, increase of body temperature, improvement of the intraoral environment, prevention of halitosis, and prevention of body odor.

### [Additional Components]

The health-promoting agent of the present invention may contain various additives as additional components, unless any of the advantageous effects of the present invention is deteriorated. For example, additives such as medicinal components, vitamins, minerals, juices, water-soluble dietary fibers, flavors, thickeners, antiseptics, sweeteners, surfactants, antioxidants, emulsifiers, coloring agents, spices, preservatives, pH adjusters, acidulants, and quality stabilizers may be blended singly or in combination.

The health-promoting agent of the present invention may be used as a food or beverage, or as a medicine. For example, the health-promoting agent of the present invention is highly useful as a functional food (e.g., a food for specified health uses, a health food, a health aid, a nutrient), since the health-promoting agent of the present invention exerts the above various beneficial functions together in combination.

The dosage form or form of the health-promoting agent of the present invention is not limited as long as the embodiment or dosage form is acceptable as a food or beverage or a medicine. In the case that the health-promoting agent of the present invention is used for foods and beverages, for example, the health-promoting agent of the present invention can be blended for use in various types of commercially available foods and beverages such as fluid diets, various types of feeds, flour products, instant foods, processed agricultural products, processed fishery products, processed livestock products, milk/dairy products, fats and oils, basic seasonings, composite seasonings/foods, frozen foods, confectioneries, and various types of beverages. In the case that the health-promoting agent of the present invention is a medicine, the health-promoting agent of the present invention can be formulated, for example, into a solid formulation such as a powder, a granule, a tablet, and a capsule; or a liquid formulation such as a solution, a syrup, a suspension, and an emulsion; a suppository; or an ointment.

The health-promoting agent of the present invention can be produced in the same manner as any known production method with known materials for foods and beverages or medicines, with addition of a step of including at least one compound as an active ingredient selected from the group consisting of the polyamine represented by formula (1), the polymer having a structural unit derived from a cyclic amine represented by formula (2), and the polymer having a structural unit derived from an unsaturated amine represented by formula (3).

### Examples

Hereinafter, the present invention will be more specifically described with reference to Examples; however, the present invention is not limited by these Examples.

### Examples 1 to 21, Comparative Examples 1 to 4

Test solutions of Examples and Comparative Examples were obtained by preparation using components listed in the table according to formulations shown in the table. Evaluations below were performed by using the test solutions shown in Table 1.

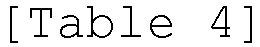

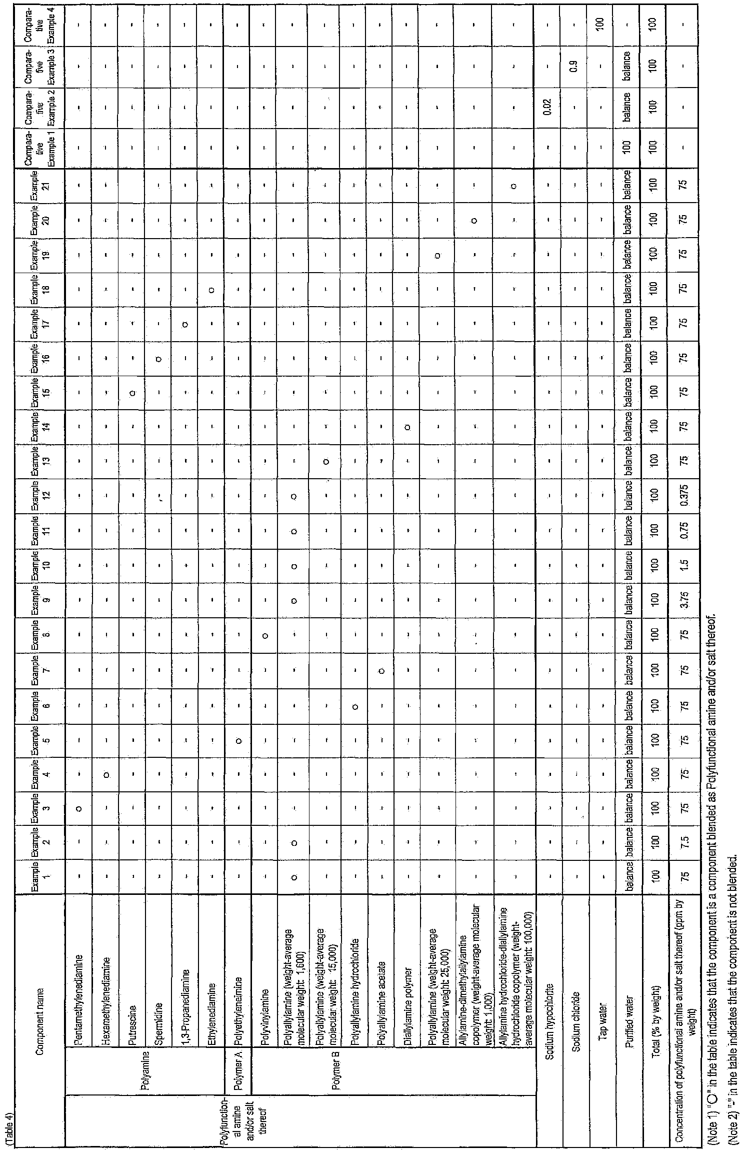

### [Test Example 1: Antimicrobial Function (1)]

To each of Examples 1 to 8 and Comparative Examples 1 to 3 (specimens), a test microorganism suspension (Escherichia coli, Staphylococcus aureus, Aspergillus niger) was seeded, and 24 hours thereafter the viable cell count in each test solution was determined.

The test conditions were as follows. Tables 5 to 7 show the results.

### (Test Microorganism Suspension)

Test microorganism 1: Escherichia coli (Escherichia coli NBRC 3972)
   Preculture: normal agar medium [Eiken Chemical Co., Ltd.], 35°C ± 1°C, 18 to 24 hours
   Solution for preparation of microorganism suspension: purified water
   Cell count: 107 to 108 cells/mL
Test microorganism 2: Staphylococcus aureus
(Staphylococcus aureus subsp. Aureus NBRC 12732)
   Preculture: normal agar medium, 35°C ± 1°C, 18 to 24 hours
   Solution for preparation of microorganism suspension: physiological saline
   Cell count: 107 to 108 cells/mL
Test microorganism 3: Aspergillus niger (Aspergillus niger NBRC 105649)
   Preculture: Potato Dextrose Agar (Difco Laboratories), 25°C ± 1°C, 7 to 10 days
   Solution for preparation of microorganism suspension: 0.005% solution of dioctyl sulfosuccinate sodium salt
   Cell count: 107 to 108 cells/mL
(Test Solution)
   Into 10 mL of each specimen, 0.1 mL of a test microorganism suspension was inoculated.
(Storage Conditions)
   24 hours (room temperature)
(Determination of Viable Cell Count)
Escherichia coli, Staphylococcus aureus: SCDLP agar medium [Nihon Pharmaceutical Co., Ltd.], pour plate culture method, 35°C ± 1°C, 2 days
Aspergillus niger: GPLP agar medium [Nihon Pharmaceutical Co., Ltd.], pour plate culture method, 25°C ± 1°C, 7 days

### [Table 5]

**(Table 5)**

| Test microorganism 1: Escherichia coli | | | | | |
|---|---|---|---|---|---|
| | Component name | Concentration (ppm by weight) | pH | Viable cell count (cells/mL) | |
| | | | | At start | After 24 hours |
| Example 1 | Polyallylamine | 75 | 9.1 | - | <10 |
| Example 2 | Polyallylamine | 7.5 | 9.8 | - | <10 |
| Example 3 | Pentamethylenediamine | 75 | 6.8 | - | <10 |
| Example 4 | Hexamethylenedlamine | 75 | 6.9 | - | <10 |
| Example 5 | Polyethylenelmine | 75 | 7.2 | - | <10 |
| Example 6 | Polyallylamine hydrochloride | 75 | 7.5 | - | 1.6×10⁴ |
| Example 7 | Polyallylamine acetate | 75 | 7.2 | - | 2.8×10⁴ |
| Example 8 | Polyvinylamine | 75 | 6.8 | - | <10 |
| Comparative Example 1 | Purified water | - | - | 5.8×10⁵ | 5.2×10⁵ |
| Comparative Example 2 | Sodium hypochlorite | 200 | 6.9 | - | <10 |

| | | | | | |
|---|---|---|---|---|---|
| * <10: not detected | | | | | |

### [Table 6]

**(Table 6)**

| Test microorganism 2: Staphylococcus aureus | | | | | |
|---|---|---|---|---|---|
| | Component name | Concentration (ppm by weight) | pH | Viable cell count (cells/mL) | |
| | | | | At start | After 24 hours |
| Example 1 | Polyallylamine | 75 | 9.1 | - | <100 |
| Example 2 | Polyallylamine | 7.5 | 9.8 | - | <10 |
| Example 3 | Pentamethylenediamine | 75 | 6.8 | - | <10 |
| Example 4 | Hexamethylenediamine | 75 | 6.9 | - | <10 |
| Example 5 | Polyethyleneimine | 75 | 7.2 | - | <1000 |
| Example 6 | Polyallylamine hydrochloride | 75 | 7.5 | - | <100 |
| Example 7 | Polyallylamine acetate | 75 | 7.2 | - | <100 |
| Example 8 | Polyvinylamine | 75 | 6.8 | - | <100 |
| Comparative Example 2 | Sodium hypochlorite | 200 | 6.9 | - | <10 |
| Comparative Example 3 | Physiological saline | - | | 4.6×10⁵ | 3.2×10⁵ |

| | | | | | |
|---|---|---|---|---|---|
| * <10, <100, and <1000: not detected | | | | | |

### [Table 7]

**(Table 7)**

| Test microorganism 3: Aspergillus niger | | | | | |
|---|---|---|---|---|---|
| | Component name | Concentration (ppm by weight) | pH | Viable cell count (cells/mL) | |
| | | | | At start | After 24 hours |
| Example 1 | Polyallylamine | 75 | 9.1 | - | 1.2×10⁵ |
| Example 2 | Polyallylamine | 7.5 | 9.8 | - | 1,1×10⁵ |
| Example 3 | Pentamethylenediamine | 75 | 6.8 | - | 4.7×10² |
| Example 4 | Hexamethylenediamine | 75 | 6.9 | - | 3.0×10² |
| Example 5 | Polyethyleneimine | 75 | 7.2 | - | 2.1×10⁵ |
| Example 6 | Polyallylamine hydrochloride | 75 | 7.5 | - | 3.7×10⁵ |
| Example 7 | Polyallylamine acetate | 75 | 7.2 | - | 3.5×10⁵ |
| Example 8 | Polyvinylamine | 75 | 6.8 | - | 2.5×10⁵ |
| Comparative Example 1 | Purified water | | - | 2.9×10⁵ | 2.4×10⁵ |
| Comparative Example 2 | Sodium hypochlorite | 200 | 6.9 | - | <10 |

| | | | | | |
|---|---|---|---|---|---|
| * <10: not detected | | | | | |

Examples 1 to 5 and 8 were found to have sterilization effect against Escherichia coli and Staphylococcus aureus. The sterilization effects of Examples 6 and 7 against Escherichia coli were relatively weak. On the other hand, Examples 1 to 8 were all found to have no effect against Aspergillus niger. Aspergillus niger is known as one of fungi universal in living environments, and is a mold that is used even in production of various types of enzyme agents and thus useful in the field of foods. Those results demonstrated that Examples 1 to 8 have selective antimicrobial function. It is suggested that such selective antimicrobial function is preferred for hygiene management of working environments in the field of foods, in particular, in the brewing industry or the like.

### [Test Example 2: Antimicrobial Function (2)]

In a sterilized plastic cup, 900 µL of each sample and 100 µL of a microorganism suspension prepared to reach 106 to 107 CFU/mL were mixed together, and left to stand. After 5, 30, and 60 minutes, 100 µL was taken from each test solution, and 10-fold diluted with 900 µL of physiological saline. This diluted solution was applied onto a standard agar medium by using a spiral spreader, culture was performed at 35°C for 48 hours, and the temporal variation of the cell count in each test solution was observed. Tables 8 to 10 show the results.

### [Table 8]

**(Table 8)**

| Test microorganism: lactic acid bacteria (Lactococcus lactis) | | | | | | |
|---|---|---|---|---|---|---|
| | Component name | Concentration (ppm by weight) | Cell count | | | |
| | | | Start (0 min) | 5 min | 30 min | 60 min |
| Example 1 | Polyallylamine | 75 | 1.6×10⁵ | <200 | <200 | <200 |
| Example 2 | Polyallylamine | 7.5 | 1.6×10⁵ | <200 | <200 | <200 |
| Comparative Example 2 | Sodium hypochlorite | 200 | 1.6×10⁵ | <200 | <200 | <200 |

### [Table 9]

**(Table 9)**

| Test microorganism: lactic acid bacteria (Leuconostoc mesenteroldes) | | | | | | |
|---|---|---|---|---|---|---|
| | Component name | Concentration (ppm by weight) | Cell count | | | |
| | | | Start (0 min) | 5 min | 30 min | 60 min |
| Example 1 | Polyallylamine | 75 | 4.5×10⁵ | <200 | <200 | <200 |
| Example 2 | Polyallyiamine | 7.5 | 4.5×10⁵ | <200 | <200 | <200 |
| Comparative Example 2 | Sodium hypochlorite | 200 | 4.5×10⁵ | <200 | <200 | <200 |

### [Table 10]

**(Table 10)**

| Test microorganism: yeast (Wickerhamomyces anomalus) | | | | | | |
|---|---|---|---|---|---|---|
| | Component name | Concentration (ppm by weight) | Cell count | | | |
| | | | Start (0 min) | 5 min | 30 min | 60 min |
| Example 1 | Polyallylamine | 75 | 1.2×10⁴ | <200 | <200 | <200 |
| Example 2 | Polyallylamine | 7.5 | 1.2×10⁴ | <200 | <200 | <200 |
| Comparative Example 2 | Sodium hypochlorite | 200 | 1.2×10⁴ | <200 | <200 | <200 |

For Examples 1 and 2 and Comparative Example 2, the cell count decreased below the detection limit after 5 minutes. It was demonstrated that Examples 1 and 2 have sterilization ability comparative to 200 ppm by weight of sodium hypochlorite (Comparative Example 1) against two lactic acid bacteria (Lactococcus lactis, Leuconostoc mesenteroides) and a yeast (Wickerhamomyces anomalus).

### [Test Example 3: Antiviral Function]

An appropriate amount of the test solution of Example 1 was sprayed on the surface of a medical surgical mask (nonwoven fabric), and effect against feline calicivirus was examined. Table 11 shows the results. For a control, purified water was used. The results found effect of decreasing the number of feline calicivirus particles attached to the surface of the nonwoven fabric.

### [Table 11]

**(Table 11)**

| Test virus: feline calicivirus | | | | | | |
|---|---|---|---|---|---|---|
| | | Concentration (ppm by weight) | log TCID50/mL | | | |
| | | | At start | After 5 min | After 30 min | After 60 min |
| Example 1 | Polyallylamine | 75 | - | <1.5 | <1.5 | <1.5 |
| Comparative Example 1 | Purified water | - | 6.7 | - | - | 7.2 |

| | | | | | | |
|---|---|---|---|---|---|---|
| TCID50: Median Tissue Culture Infections Dose Operative temperature: room temperature | | | | | | |

The test solution of Example 1 was found to have effect of decreasing the number of feline calicivirus particles attached to the nonwoven fabric. For Comparative Example 1, effect of decreasing the number of feline calicivirus particles was not found.

### [Test Example 4: Rust-Preventing Function]

Two or three Gem clips (major axis: 25 mm) made of iron (with nickel plating) were disposed in each of flat containers with a diameter of approximately 5 cm and a depth of 1 cm, and the test solutions of Examples 1, 3 to 5, 7, and 13 to 18, and Comparative Example 1 were then poured into the respective containers, completely soaking the Gem clips therewith. The state of each Gem clip was visually observed after a lapse of 7 days from the start of the test, and rust-preventing function was evaluated on the basis of the following criteria. Table 12 shows the results.

### <Evaluation Criteria for Rust-Preventing Function>

∘: The generation of rust was not found.
×: The generation of rust was found.

### [Table 12]

**(Table 12)**

| | Component name | Concentration (ppm by weight) | Result |
|---|---|---|---|
| Example 1 | Polyallylamine (weight-average molecular weight: 1,600) | 75 | ○ |
| Example 3 | Pentamethylenediamine | 75 | ○ |
| Example 4 | Hexamethylenediamine | 75 | ○ |
| Example 5 | Polyethyleneimine | 75 | ○ |
| Example 7 | Polyallylamine acetate | 75 | ○ |
| Example 13 | Polyallylamine (weight-average molecular weight: 15,000) | 75 | ○ |
| Example 14 | Diallylamine polymer | 75 | ○ |
| Example 15 | Putrescine | 75 | ○ |
| Example 16 | Spermidine | 75 | ○ |
| Example 17 | 1,3-Propanediamine | 75 | ○ |
| Example 18 | Ethylenediamine | 75 | ○ |
| Comparative Example 1 | Purified water | - | × |

The generation of rust was found for Comparative Example 1, but the generation of rust was not found at all for Examples 1, 3 to 5, 7, and 13 to 18.

### [Test Example 5: Freshness-Keeping Function for Fish, Aging Function for Fish]

Prepared was 1000 mL of each of the test solutions of Example 1 and Comparative Example 1, and three raw, whole jack mackerels were soaked in each test solution for 10 minutes. Thereafter, the jack mackerels were taken from each test solution, and each covered with a plastic wrap, and stored in a refrigerator for 3 days.

First, three Japanese food cooks smelled each of the raw jack mackerels treated with Example 1 or Comparative Example 1 before being cooked, and evaluated on the basis of the following criteria on whether fishiness, as an indicator of freshness, was prevented with reference to the raw jack mackerels treated with Comparative Example 1.

### <Evaluation Criteria for Fishiness>

5 points: There was completely no fishiness as compared with the case with Comparative Example 1.
3 points: There was almost no fishiness as compared with the case with Comparative Example 1.
1 point: There was fishiness comparable to that in the case with Comparative Example 1.

Subsequently, the raw jack mackerels were each roasted on a frying pan, and three Japanese food cooks then ate them, and evaluated on the basis of the following evaluation criteria on whether meat quality was retained with reference to the roasted jack mackerels obtained by cooking the raw jack mackerels treated with Comparative Example 1.

### <Evaluation Criteria for Retention of Meat Quality>

5 points: The fish meat gave completely no dry feeling as compared with the case with Comparative Example 1, and was very juicy in terms of meat quality.
3 points: The fish meat gave almost no dry feeling as compared with the case with Comparative Example 1, and was juicy in terms of meat quality.
1 point: The fish meat gave dry feeling comparable to that in the case with Comparative Example 1, and was not juicy in terms of meat quality.

Average scores were calculated from the points determined by the three cooks, and scores of 3.5 points or more were rated as "⊙" (excellent), scores of less than 3.5 points and 2.5 points or more as "∘" (practically acceptable), and scores of less than 2.5 points as "×" (poor), and comprehensive evaluation was performed. Table 13 shows the results.

### [Table 13]

**(Table 13)**

| Food: raw, whole jack mackerels | | |
|---|---|---|
| | Fishiness | Retention of meat quality |
| Cook A | 5 points | 3 points |
| Cook B | 5 points | 5 points |
| Cook C | 3 points | 5 points |
| Average score | 4.3 points | 4.3 points |
| Comprehensive evaluation | ⊙ (4.3 points) | |

The jack mackerels treated with Example 1 did not generate any fishiness even after the lapse of 3 days, and were evaluated to have meat quality kept good, and thus Example 1 was demonstrated to be superior in freshness-keeping function. Surprisingly, a comment that the jack mackerels treated with Example 1 had enhanced umami was given. It is inferred that treatment with Example 1 promoted aging to enhance umami.

### [Test Example 6: Freshness-Keeping Function for Animal Meat, Aging Function for Animal Meat]

Sheets of commercially available meat paper was saturated with each of the test solutions of Example 1 and Comparative Example 1, and three blocks of 100 g of American beef tenderloin for steak were each wrapped with the sheet of meat paper and stored in a refrigerator for 7 days. After 7 days, the blocks of American beef tenderloin for steak treated with Example 1 and those treated with Comparative Example 1 were each roasted on a frying pan, and three western food cooks then ate them, and evaluated then on the basis of the following criteria on color change/odor, change of taste, and whether the meat quality was retained, with reference to the cooked blocks of American beef tenderloin for steak treated with Comparative Example 1.

### <Evaluation Criteria for Color Change/Odor and Change of Taste>

5 points: There was completely no color change or odor as compared with the case with Comparative Example 1, with the taste being as expected.
3 points: There was almost no color change or odor as compared with the case with Comparative Example 1, with the taste being as expected.
1 point: There was color change and odor as compared with the case with Comparative Example 1, with the taste not being as expected.

### <Evaluation Criteria for Retention of Meat Quality>

5 points: The steak gave completely no dry feeling as compared with the case with Comparative Example 1, and was very juicy in terms of meat quality.
3 points: The steak gave almost no dry feeling as compared with the case with Comparative Example 1, and was juicy in terms of meat quality.
1 point: The steak gave dry feeling comparable to the case with Comparative Example 1, and was not juicy in terms of meat quality.

Average scores were calculated from the points determined by the three cooks, and scores of 3.5 points or more were rated as "⊙" (excellent), scores of less than 3.5 points and 2.5 points or more as "∘" (practically acceptable), and scores of less than 2.5 points as "×" (poor), and comprehensive evaluation was performed. Table 14 shows the results.

### [Table 14]

**(Table 14)**

| Food: American beef tenderloin for steak | | |
|---|---|---|
| | Change of color/odor, change of taste | Retention of meat quality |
| Cook A | 3 points | 5 points |
| Cook B | 3 points | 5 points |
| Cook C | 5 points | 3 points |
| Average score | 3.7 points | 4.3 points |
| Comprehensive evaluation | ⊙ (4 points) | |

The American beef tenderloin for steak treated with Example 1 had neither color change nor odor even after the lapse of 7 days, and gave steaks with taste as expected therefor and the steaks were juicy in terms of meat quality. These results demonstrated superior freshness-keeping function for animal meat. Surprisingly, a comment that the steaks of American beef tenderloin treated with Example 1 had enhanced umami. It is inferred that treatment with Example 1 promoted aging to enhance umami.

### [Test Example 7: Freshness-Keeping Function for Fruits]

Evaluated was freshness-keeping function for fruits stored in water, in which release of ethylene gas was suppressed. Unpeeled bananas without any sugar spot (brown spot) were each sealed with a plastic wrap, and the bananas were respectively soaked in the test solutions of Example 1 and Comparative Example 1 for 14 days. Thereafter, the unpeeled bananas were taken out of the test solutions, and the appearance of each banana peel was evaluated by visual observation on the basis of the following evaluation criteria.

### <Evaluation Criteria for Banana Peel>

∘: The area occupied by brown spots in the banana peel was less than 30%.
Δ: The area occupied by brown spots in the banana peel was not less than 30% and less than 60%.
×: The area occupied by brown spots in the banana peel was 60% or more.

The appearance for Example 1 was rated as "o", and the appearance for Comparative Example 1 was rated as "Δ". These results demonstrated that Example 1 has function of further keeping freshness of bananas under storage conditions with generation of ethylene gas suppressed.

### [Test Example 8: Antiseptic Function 1]

### (1) Cabbage

On April 11, 2008, a glass bottle containing approximately 200 mL of the test solution of Example 1 was prepared, and approximately 10 cut pieces obtained by cutting a cabbage leaf into pieces of appropriate size were soaked in the test solution, and the glass bottle was covered with a lid and left to stand. As of January 2019, the cabbage leaf was free from decomposition and there was no odor. In addition, the shape was retained almost in the original shape.

As a control, the same experiment was conducted except that tap water was used in place of the test solution of Example 1, and the result was that the cabbage leaf began to decompose on about April 20, 2008, and had become like a sludge and decomposed and dissolved in water before June 15 in the same year.

### (2) Podded Pea

On April 11, 2008, a glass bottle containing approximately 200 mL of the test solution of Example 1 was prepared, and three podded peas were soaked in the test solution, and the glass bottle was covered with a lid and left to stand. As of January 2019, the podded peas were free from decomposition and there was no odor. In addition, the shape was retained almost in the original shape.

As a control, the same experiment was conducted except that tap water was used in place of the test solution of Example 1, and the result was that the podded peas began to decompose on about April 20, 2008, and had become like a sludge and decomposed and dissolved in water before June 15 in the same year.

### (3) Slice of fish (longtooth grouper)

On April 11, 2008, a glass bottle containing approximately 200 mL of the test solution of Example 1 was prepared, and a slice of fish (longtooth grouper) was soaked in the test solution, and the glass bottle was covered with a lid and left to stand. As of January 2019, the slice of fish was free from decomposition and there was no odor. In addition, the shape was retained almost in the original shape.

As a control, the same experiment was conducted except that tap water was used in place of the test solution of Example 1, and the result was that the slice of fish began to decompose on about April 14, 2008, and had become like a sludge and decomposed and dissolved in water before April 20 in the same year.

### (4) Dead goldfish

On March 16, 2013, a glass bottle containing approximately 1000 mL of the test solution of Example 1 was prepared, and a goldfish (body length: approximately 17 cm) that had just died was soaked in the test solution, and the glass bottle was covered with a lid and left to stand. As of January 2019, the goldfish was free from decomposition and there was no odor. In addition, the shape was retained almost in the original shape.

As a control, the same experiment was conducted except that tap water was used in place of the test solution of Example 1, and the result was that the goldfish began to decompose on about March 20, 2013, and had become like a sludge and decomposed and dissolved in water before April 28 in the same year.

### [Test Example 9: Deodorizing Function]

The deodorizing function of Example 1 was evaluated under the following conditions. Table 15 shows the results.
Test method: detector tube method
Test container: 5 L smart bag PA
Gas volume in container: 3 L
Gas measurement method: detector tube
Initial gas concentration:
   ammonia 100 ppm
   hydrogen sulfide 4 ppm
   trimethylamine 28 ppm
Test sample: Example 1 was sprayed on a sheet of commercially available paper, and the saturated sheet was used.
Control:
   blank test (the same operation was performed without use of the sample)
   water test (water was sprayed on a sheet of commercially available paper, and the saturated sheet was used)
Measurement time: after 1 hour and after 2 hours

### [Table 15]

**(Table 15)**

| Deodorizing function | | | | |
|---|---|---|---|---|
| | Measurement time | Concentration (ppm). | | |
| | | Example 1 | Blank test | Water test |
| Ammonia | After 1 hour | 6.1 | 91.3 | 90.5 |
| | After 2 hours | 4.0 | 85.7 | 84.7 |
| Hydrogen sulfide | After 1 hour | 2.6 | 4.0 | 4.0 |
| | After 2 hours | 1.9 | 4.0 | 4.0 |
| Trimethylamine | After 1 hour | 5.1 | 28.0 | 28.0 |
| | After 2 hours | 2.9 | 28.0 | 28.0 |

Example 1 was demonstrated to have deodorizing function for any of ammonia, hydrogen sulfide, and trimethylamine.

### [Test Example 10: Life-Extending Function 1 for Cut Flowers (Rose)]

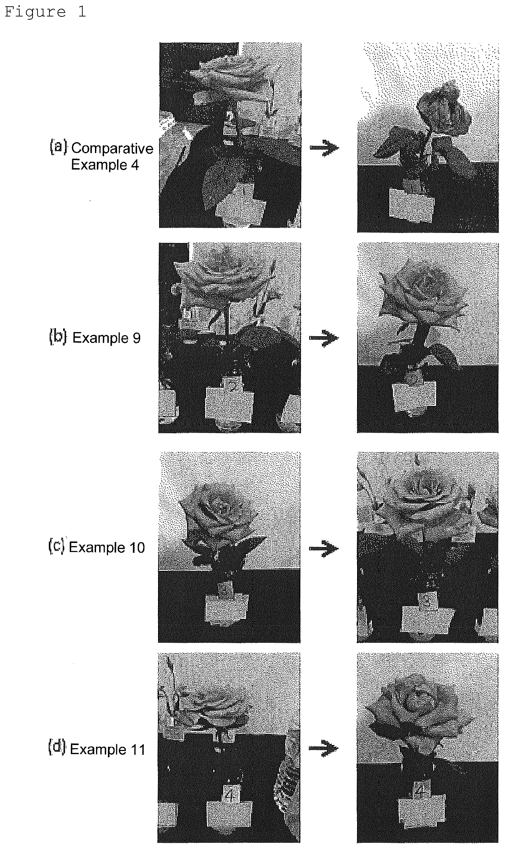

The test solutions of Examples 9 to 11 and Comparative Example 4 were prepared. Roses were used as cut flowers, and each rose was placed in a container in such a manner that the cut end was soaked in the test solution. The roses were left to stand under such conditions that the roses were irradiated with room light (fluorescent light) for approximately 10 hours in a day at a temperature of 20 ± 3°C. The state of each rose 4 days after the start of the test was visually observed. During the test period, exchange of the test solutions was not performed. Figure 1 shows the results.

Withering was found for the rose with Comparative Example 4, but no change was found for the roses with Examples 9 to 11. In comparing Examples 9 to 11, life-extending function was confirmed at any concentration. The functional water of the present invention has antimicrobial function and suppresses the proliferation unwanted microorganisms that clog vessels of plants, and hence is inferred to advantageously act on life-extending for cut flowers.

### [Test Example 11: Life-Extending Function 2 for Cut Flowers (Texas Bluebell: Part 1)]

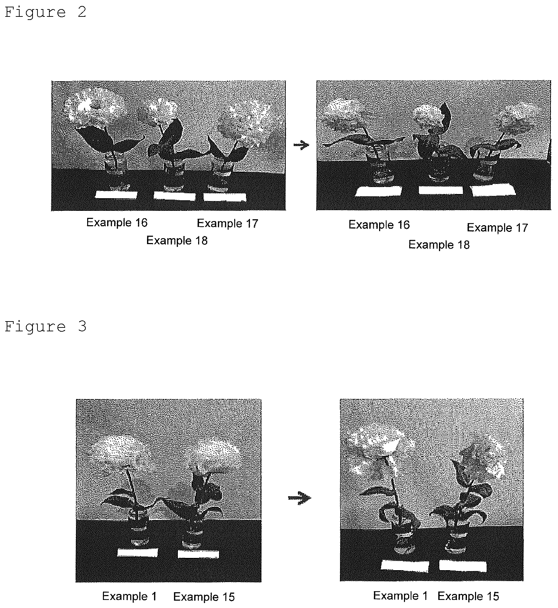

The test solutions of Examples 16 to 18 were prepared. Texas bluebells were used as cut flowers, and each Texas bluebell was placed in a container in such a manner that the cut end was soaked in the test solution. The Texas bluebells were left to stand under such conditions that the Texas bluebells were irradiated with room light (fluorescent light) for approximately 10 hours in a day at a temperature of 20 ± 3°C. The state of each Texas bluebell 15 days after the start of the test was visually observed. During the test period, exchange of the test solutions was not performed. Figure 2 shows the results.

For all of Examples, life-extending effect was found.

### [Test Example 12: Life-Extending function 3 for Cut Flowers (Texas Bluebell: Part 2)]

The test solutions of Examples 1 and 13 to 15 were prepared. Texas bluebells were used as cut flowers, and each Texas bluebell was placed in a container in such a manner that the cut end was soaked in the test solution. The Texas bluebells were left to stand under such conditions that the Texas bluebells were irradiated with room light (fluorescent light) for approximately 10 hours in a day at a temperature of 20 ± 3°C. The state of each Texas bluebell 11 days after the start of the test was visually observed. During the test period, exchange of the test solutions was not performed. Figure 3 shows the results for Example 1 and Example 15.

For all of Examples, life-extending effect was found. The Texas bluebell soaked in Example 1 maintained better flowering state than those with Examples 13 and 14 (not shown) and 15.

### [Test Example 13: Flowering-Adjusting Function 1 for Cut Flowers (Texas Bluebell)]

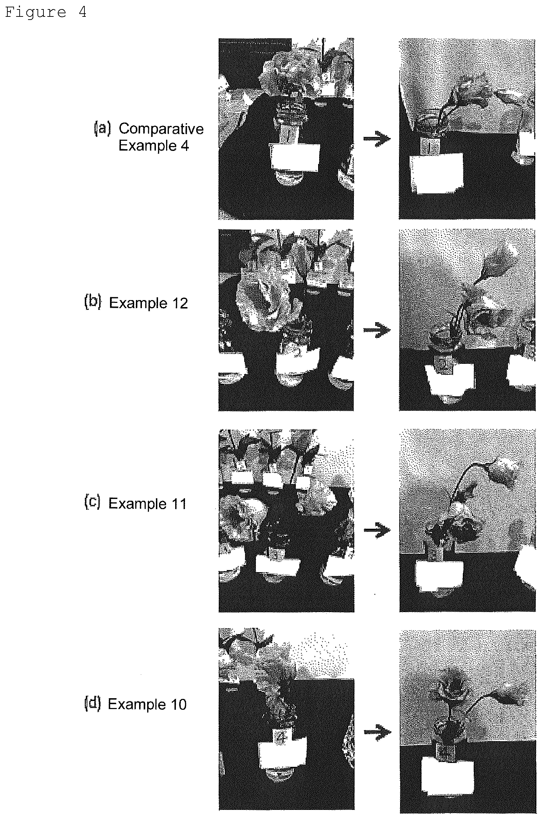

The test solutions of Examples 10 to 12 and Comparative Example 4 were prepared. Texas bluebells with a bud and a flower were used as cut flowers, and each Texas bluebell was placed in a container in such a manner that the cut end was soaked in the test solution. The Texas bluebells were left to stand under such conditions that the Texas bluebells were irradiated with room light (fluorescent light) for approximately 10 hours in a day at a temperature of 20 ± 3°C. The state of each bud and flower 5 days after the start of the test was visually observed. During the test period, exchange of the test solutions was not performed. Figure 4 shows the results.

With Comparative Example 4, the flower withered and the bud did not open. By contrast, the buds with Examples 10 and 12 were in 50% bloom, and the bud with Example 11 was in 80% bloom. Moreover, the flowers with Examples 10 to 12 did not wither.

### [Test Example 14: Flowering-Adjusting Function 2 for Cut Flowers (Lily)]

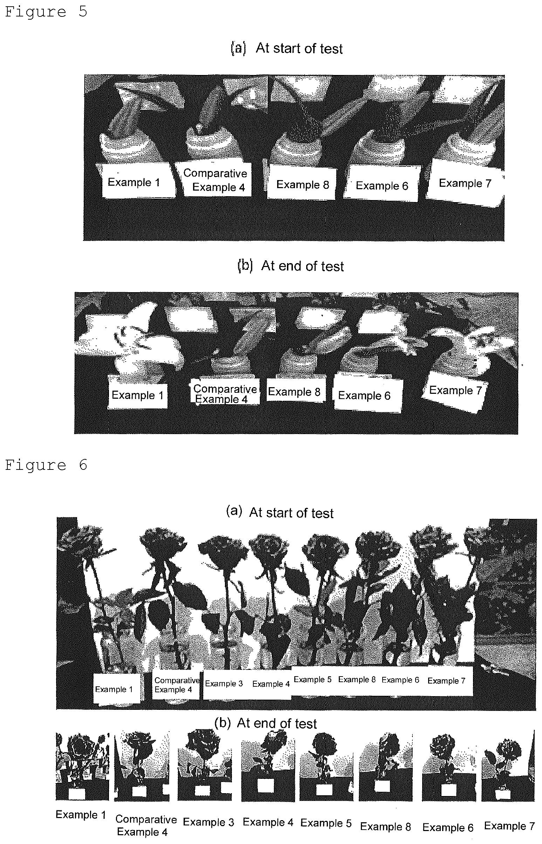

The test solutions of Examples 1 and 6 to 8 and Comparative Example 4 were prepared. Lily buds were used as cut flowers, and each lily was placed in a container in such a manner that the cut end was soaked in the test solution. The lilies were left to stand under such conditions that the lilies were irradiated with room light (fluorescent light) for approximately 10 hours in a day at a temperature of 20 ± 3°C. The state of each lily bud 12 days after the start of the test was visually observed. During the test period, exchange of the test solutions was not performed. Figure 5 shows the results.

The lily buds with Examples 1 and 7 completely opened. The bud with Example 6 was in 50% bloom, and the bud with Example 8 was in 30% bloom. The bud with Comparative Example 4 did not open.

### [Test Example 15: Flowering-Adjusting Function 3 for Cut Flowers (Rose)]

The test solutions of Examples 1 and 3 to 8 and Comparative Example 4 were prepared. Commercially available roses were used as cut flowers, and each rose was placed in a container in such a manner that the cut end was soaked in the test solution. The roses were left to stand under such conditions that the roses were irradiated with room light (fluorescent light) for approximately 10 hours in a day at a temperature of 20 ± 3°C. The state of each rose 10 days after the start of the test was visually observed. During the test period, exchange of the test solutions was not performed. Figure 6 shows the results.

The rose with Comparative Example 4 did not undergo further progression of opening from the start of the test. By contrast, the roses with Examples 1 and 3 to 8 were all found to undergo progression of opening from the start as a trend. The rose with Example 1 was keeping the best flowering state after 10 days. The roses with Examples 4 and 8 underwent progression of opening during the test period, and the necks were found to slightly bend at the end of the test. This is inferred to be due to the effect of the concentration of the polyfunctional amine (possibly, due to high concentration).

### [Test Example 16: Plant-Growth-Adjusting Function 1 (Pea Sprout)]

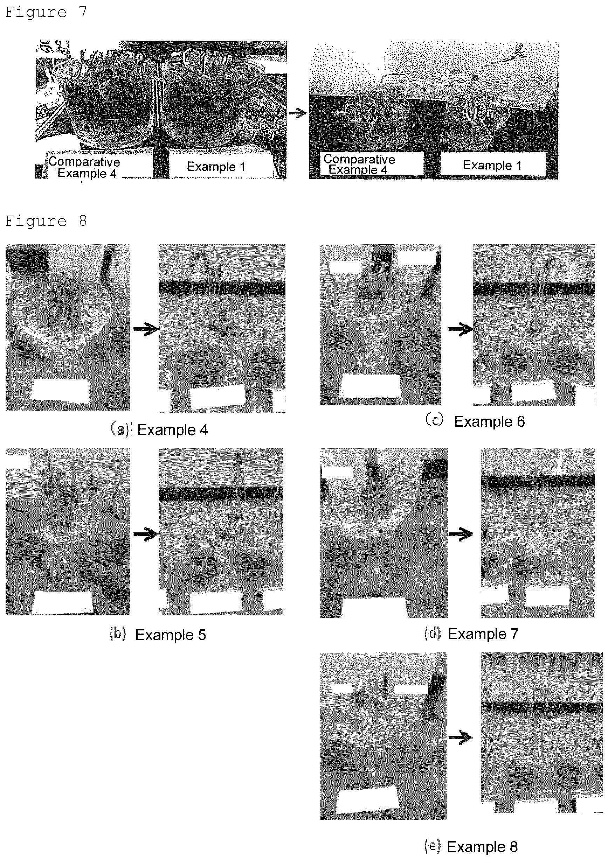

The test solutions of Examples 1 and 3 to 8 and Comparative Example 4 were prepared. Commercially available pea sprouts were purchased, and each pea sprout was cut at about 2 cm from the end of the root to remove the upper part, and the pea sprouts were each soaked in the corresponding test solution. The pea sprouts were left to stand under such conditions that the pea sprouts were irradiated with room light (fluorescent light) for approximately 10 hours in a day at a temperature of 20 ± 3°C. The regeneration rate (regeneration from a lateral bud) of each pea sprout 9 days after the start of the test was visually observed. During the test period, exchange of the test solutions was not performed. Figures 7 and 8 show the results.

The regeneration rates of pea sprouts with Examples 1 and 3 (not shown) and 4 to 8 were higher than that with Comparative Example 4. In addition, growth of lateral buds was more rapid in the pea sprouts with Examples. While it is needed in common regenerative cultivation of pea sprouts to exchange water every day in order to prevent decomposition of peas, gathering of mold, and generation of odor, the test waters of Examples have antimicrobial function, and hence can be expected to reduce time of water exchange.

### [Test Example 17: Plant-Growth-Adjusting Function 2 (Sprouting of Soybean)]

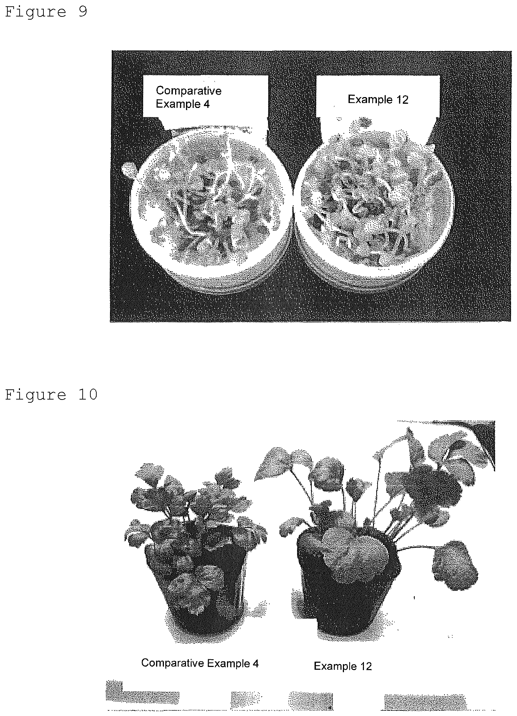

The test solutions of Example 12 and Comparative Example 4 were prepared. A gauze was laid on the inner bottom of each of cylindrical containers, and 30 soybeans soaked in water were seeded on each gauze. Thereafter, each test solution was poured to submerge the soybeans in the container. The soybeans were left to stand in a room at a temperature of 23°C and a humidity of 40% for 7 days. The condition of sprouting after 7 days was visually observed. Figure 9 shows the results.

The soybeans soaked in Example 12 exhibited a better sprouting rate and more rapid sprout growth than the soybeans soaked in Comparative Example 4. Example 12 (dose of polyfunctional amine: 0.375 ppm by weight) was demonstrated to have promoting function for sprouting and rooting.

### [Test Example 18: Plant-Growth-Adjusting Function 3 (Persian Buttercup)]

The test solutions of Example 12 and Comparative Example 4 were prepared. Two seedlings of Persian buttercup each put in a seedling pot were prepared. Each test solution was sprinkled to such a degree that the soil was moistened, and the seedlings were grown outside (in a balcony). The condition of growth of each Persian buttercup after 14 days was visually observed. Figure 10 shows the results.

The growth of the seedling of Persian buttercup with sprinkling of Example 12 (amount of polyfunctional amine: 0.375 ppm by weight) was clearly promoted as compared with the seedling of Persian buttercup with sprinkling of Comparative Example 4. In particular, the surface area of leaves and stem height were both increased approximately 1.5 times or more through the growth.

### [Test Example 19: Pest-Controlling Function (Two-Spotted Spider Mite, Aphid, Scale Insect)]

The pest-control function of the functional water of the present invention is inferred to be caused by one of suffocation effects due to the smallness of clusters of water. Hence, difference in evaporation time (evaporation speed) between the functional water of the present invention and purified water was evaluated as a preliminary test. This was on the assumption that smaller clusters of water facilitate fly-out of water molecules from a water surface, and thus fast evaporation will be observed in evaluation.

### Preliminary Test: Examination of Smallness of Water Clusters in Functional Water

Prepared were 100 mL of the test solution of Comparative Example 1 and that of Example 1. Each test solution was put in a beaker, and heated with an electromagnetic cooker under conditions below, and time until complete evaporation was achieved was measured with a stopwatch. At this time, the state of each liquid surface at boiling was visually observed.
Instrument used: National electromagnetic cooker IH 3.0 kW
State in use: Memory 5 → higher middle
Test result:
   Comparative Example 1: 5 min 45 sec 73
   Example 1: 5 min 39 sec 98

The result of the preliminary test showed that the test solution of Example 1 evaporated faster than the test solution of Comparative Example 1. The test solution of Example 1 did not splash at all, but 100 or more water droplets flying out were found at around the boiling point for Comparative Example 1, and hence it was needed to retest with attachment of a splashing-preventing frame.

### Main Test 1: Pest-Controlling Function (Two-Spotted Spider Mite)

Pest-controlling function against two-spotted spider mites was evaluated in the following procedure.
1. Two-spotted spider mites purchased from SUMIKA TECHNOSERVICE CORPORATION were placed on a glass slide (approximately 25 mm × 75 mm).
2. A commercially available spray container was filled with the test solution of Example 1, and the test solution was sprayed to the two-spotted spider mites on the glass slide to such a degree that the bodies got wet.
3. Through observation of movements of the two-spotted spider mites with a microscope (model name: "DO·nature STV-451M", produced by KenkoTokina Corporation, magnification: 200×), time until the movements ceased was measured with a stopwatch.

The movements of the two-spotted spider mites to which the test solution of Experiment Example 1 had been sprayed ceased in approximately 30 seconds. Observation was continued thereafter, but restart of movement was not found even after the water droplets were dried out. Observation with magnification found that fine water droplets of the test solution of Example 1 were attaching to the surface of each two-spotted spider mite. Because it is inferred from the result of the preliminary test that water clusters in the functional water of the present invention are smaller than those in purified water, the pest-controlling function is inferred to be due to the phenomenon that water droplets of the functional water of the present invention clog spiracles of two-spotted spider mites to cause suffocation.

The main test using tap water in place of the test solution of Example 1 was conducted as a control, and the result found that movements of two-spotted spider mites did not ceased.

### Main Test 2: Pest-Controlling Function (Aphid)

Pest-controlling function against aphids was evaluated in the following procedure.

A commercially available spray container was filled with the test solution of Example 1, and the test solution was sprayed to about 10 aphids parasitizing a rose. The condition of survival was checked after 1 hour. The result found that the movements of all the aphids ceased and thus they were killed. Since then, restart of movement had not been found even after the water droplets were dried out.

Tap water was sprayed as a control in place of the test solution of Example 1, but no aphid died.

### Main Test 3: Pest-Controlling Function (Scale Insect)

A commercially available spray container was filled with the test solution of Example 1, and the test solution was sprayed to several scale insects parasitizing a Japanese apricot tree. The condition of survival was checked after 1 hour. The result found that the movements of all the scale insects ceased and thus they were killed. Since then, restart of movement had not been found even after the water droplets were dried out.

Tap water was sprayed as a control in place of the test solution of Example 1, but no scale insect died.

### [Test Example 20: Parasite-Preventing Function 1 (Anisakis)]

Several Anisakis nematodes were collected from internal organs of several young yellowtails. The Anisakis nematodes collected were placed in a Petri dish, and the test solution of Example 1 was poured therein. As a result, the movements of the Anisakis nematodes ceased in approximately 5 seconds. Since then, restart of movement had not been found even after the water droplets were dried out.

Tap water was sprayed as a control in place of the test solution of Example 1, but no Anisakis nematode died.

### [Test Example 21: Parasite-Preventing Function 2 (Anisakis)]

A saku (a block of fish meat) of a young yellowtail was soaked in the test solution of Example 1 filling a container. After 24 hours, Anisakis nematodes crawling out of the saku (a block of fish meat) were found. The test solution of Example 1, which has antimicrobial function, food preservation function, aging function, and so on, was further revealed to have parasite-preventing function in combination. In particular, it is suggested that the test solution of Example 1 is extremely useful for pretreatment of fish and shellfish for raw consumption such as sashimi.

### [Test Example 22: Evaluation of Safety 1]

A single dose oral toxicity test for rats with the test solution of Example 1 was conducted by an external testing laboratory with use of five female rats and five male rats with reference to the Guidelines for Toxicity Studies of Drugs. Example 1 was directly administered at a dose of 20 mL/kg. The result found neither death nor abnormality of general condition during the observation period for 14 days. Transition of body weight during the observation period was favorable in all of the cases, and no abnormality was found even in necropsy. From these results, single administration of the test solution of Example 1 in a dose of 20 mL/kg is determined to exhibit no toxicity to rats.

### [Test Example 23: Evaluation of Safety 2]

From 2 L of commercially available mineral water in a container of a plastic bottle, 10 mL was taken, and 10 mL of the test solution of Example 1 was added thereto for inclusion. Water quality examination was conducted for the resulting specimen (concentration of polyfunctional amine: 0.375 ppm by weight) to find that the specimen met the criteria for "water suitable for drinking (water for food production)" (referring to water meeting 26 criteria) based on the Food Sanitation Act. It was confirmed that if the test solution of Example 1 is used as a material for preparing the functional water of the present invention and diluted at an arbitrary dilution rate, the diluted product is drinkable with safety as water for food production or bottled water.

### [Test Example 24: Bowel Movement Test (Bowel- Movement-Improving Function, Fecal-Odor-Reducing Function)]

Five healthy test subjects A to E (three males, two females, average age: 56 years old) were allowed to freely drink the test solution of Example 1 so as to take an absolute dose of 30 mL/day (dose of polyfunctional amine: 2.25 mg/day). Variation of fecal volume from day 1 to day 14 after the start of the test was recorded. The amounts of meals and exercise were as usual, except that the test solution of Example 1 was ingested. The condition of bowel movement was evaluated in self-assessment questionnaire based on the following criteria. Table 16 shows the test results.

### [Evaluation Results]

⊙: With defecation (fecal volume: about 1.5 to 2 times)
∘: With defecation (fecal volume: normal)
Δ: With defecation (fecal volume: small)
×: Without defecation

### [Table 16]

**(Table 16)**

| | Sex | Age | Day 1 | Day 2 | Day 3 | Day 4 | Day 5 | Day 6 | Day 7 | Day 8 | Day 9 | Day 10 | Day 11 | Day 12 | Day 13 | Day 14 |
|---|---|---|---|---|---|---|---|---|---|---|---|---|---|---|---|---|
| Test subject A | male | 71 | ○ | ○ | ○ | ○ | ⊙ | ⊙ | ⊙ | ⊙ | ⊙ | ⊙ | ⊙ | ⊙ | ⊙ | ⊙ |
| Test subject B | female | 67 | x | × | Δ | × | Δ | ○ | ○ | ○ | ○ | ○ | ○ | ○ | ○ | ○ |
| Test subject C | male | 44 | ○ | ○ | ○ | ○ | ○ | ○ | ○ | ○ | ⊙ | ⊙ | ⊙ | ⊙ | ⊙ | ⊙ |
| Test subject D | female | 33 | × | Δ | × | Δ | Δ | Δ | ○ | Δ | ○ | Δ | ○ | ○ | ○ | ○ |
| Test subject E | male | 65 | ○ | ○ | ○ | ○ | ○ | ○ | ○ | ⊙ | ⊙ | ⊙ | ⊙ | ⊙ | ⊙ | ⊙ |

Test subject B and test subject D had no defecation or infrequent defecation as a trend from the start of the test to day 4, but defecation once per day, although the fecal volume was small, was found from around day 5 after the beginning of drinking the test solution of Example 1, and defecation with normal fecal volume continued after day 6 until the end of the test. On the other hand, test subject A, test subject C, and test subject E found defecation with normal volume from the start of the test, but fecal volume about 1.5 to 2 times larger was appeared as a trend from day 5, and this increased defecation volume continued until the end of the test. From these results, it is inferred that intestinal condition and thus bowel movement in each subject were improved by the test solution of Example 1. In addition, a comment that fecal odor was reduced in association with improvement of bowel movement was reported from the test subjects.

### [Test Example 25: Blood-Pressure-Reducing Function]

Five healthy test subjects A to E (three males, two females, average age: 56 years old) were allowed to freely drink the test solution of Example 1 so as to take an absolute dose of 30 mL/day (dose of polyfunctional amine: 2.25 mg/day). The first time of the beginning of drinking in a day was 7 o'clock in the morning. Systolic blood pressure (at 9 o'clock, 12 o'clock, 20 o'clock) 1 day before the start of the test and systolic blood pressure (at 9 o'clock, 12 o'clock, 20 o'clock) after 14-day drinking were compared to evaluate blood-pressure-reducing function. Table 17 shows the results.

### [Table 17]

**(Table 17)**

| Blood-pressure-reducing function | | | | | | | | |
|---|---|---|---|---|---|---|---|---|
| Test subject | Sex | Age | Blood pressure 1 day before start | | | Blood pressure after 2 weeks | | |
| | | | 9 o'clock | 12 o'clock | 20 o'clock | 9 o'clock | 12 o'clock | 20 o'clock |
| Test subject A | male | 71 | 136 | 140 | 133 | 129 | 135 | 128 |
| Test subject B | female | 67 | 140 | 142 | 138 | 135 | 134 | 127 |
| Test subject C | male | 44 | 129 | 128 | 123 | 120 | 118 | 120 |
| Test subject D | female | 33 | 133 | 135 | 128 | 130 | 128 | 120 |
| Test subject E | male | 65 | 142 | 145 | 140 | 133 | 138 | 129 |
| | Average | 56 | 136 | 138 | 132.4 | 129.4 | 130.6 | 124.8 |

All of the test subjects were found to undergo reduction of systolic blood pressure at each time after 14-day drinking, where the reduction was 11 mmHg at the maximum and 3 mmHg at the minimum. No case with increased blood pressure was found. The average value of blood pressure after 14-day drinking decreased to around the "normal blood pressure level (systolic blood pressure): 120 to 129" specified in "Guidelines for the Management of Hypertension 2014" published by the Japanese Society of Hypertension, and the blood-pressure-reducing function of the functional water of the present invention was proved.

### [Test Example 26: Urinary-Glucose-Reducing Function]

Five healthy test subjects A to E (three males, two females, average age: 56 years old) were allowed to freely drink the test solution of Example 1 so as to take an absolute dose of 30 mL/day (dose of polyfunctional amine: 2.25 mg/day). The first time of the beginning of drinking in a day was 7 o'clock in the morning, and the test subjects were allowed to freely ingest until 6 o'clock in the evening. Each test subject collected urine 1 hour after a meal, soaked the urine in a commercially available diagnostic agent for urinary glucose (product name: "New Uriace Ga", produced by Terumo Corporation) for 1 second, and performed self-check of the glucose concentration of the urine through comparison of the color of the diagnostic agent with a color chart. Table 18 shows the results.

### [Table 18]

**(Table 18)**

| Urinary-glucose-reducing function | | | | |
|---|---|---|---|---|
| Test subject | Sex | Age | Urinary glucose level at start | Urinary glucose level after 14 days |
| | | | Unit: mg/dL | Unit: mg/dL |
| Test subject A | male | 71 | 100 | 50 |
| Test subject B | female | 67 | 50 | 50 |
| Test subject C | male | 44 | 0 | 0 |
| Test subject D | female | 33 | 0 | 0 |
| Test subject E | male | 65 | 50 | 0 |
| Average value | | | 40 | 20 |

A slight amount of urinary glucose was detected only for test subject A at the start of the test (100 mg/dL), but almost no urinary glucose was detected after 14 days (50 mg/dL). Urinary glucose test is an index used as screening for diabetes mellitus, and the functional water of the present invention was demonstrated to have function of reducing urinary glucose and to be useful for health promotion in individuals with high glucose level.

### [Test Example 27: Body-Temperature-Increasing Function (After Drinking)]

Five healthy test subjects A to E (three males, two females, average age: 56 years old) were allowed to freely drink the test solution of Example 1 so as to take an absolute dose of 30 mL/day (dose of polyfunctional amine: 2.25 mg/day). The first time of the beginning of drinking in a day was 7 o'clock in the morning, and the test subjects were allowed to freely ingest until 6 o'clock in the evening. Body temperature (at 9 o'clock, 12 o'clock, 20 o'clock) 1 day before the start of the test and body temperature (at 9 o'clock, 12 o'clock, 20 o'clock) after 14-day drinking were compared to evaluate body-temperature-increasing function. Table 19 shows the results.

### [Table 19]

**(Table 19)**

| Body-temperature-increasing function (after drinking) | | | | | | | | |
|---|---|---|---|---|---|---|---|---|
| Test subject | Sex | Age | Body temperature 1 day before start | | | Body temperature after 2 weeks | | |
| | | | 9 o'clock | 12 o'clock | 20 o'clock | 9 o'clock | 12 o'clock | 20 o'clock |
| Test subject A | male | 71 | 36.1 | 36.3 | 36.2 | 36.3 | 36.5 | 36.5 |
| Test subject B | female | 67 | 35.8 | 36.1 | 36.1 | 36.2 | 36.4 | 36.5 |
| Test subject C | male | 44 | 36.3 | 36.5 | 36.4 | 36.6 | 36.6 | 36.7 |
| Test subject D | female | 33 | 36.1 | 36,2 | 36.1 | 36.4 | 36.5 | 36.5 |
| Test subject E | male | 65 | 36.2 | 36.5 | 36.3 | 36.6 | 36.6 | 36.7 |
| | Average | 56 | 36.1 | 36.32 | 36.22 | 36.42 | 36.52 | 36,58 |

It was found that body temperature at each measurement point increased by approximately 0.3°C on average through 14-day drinking.

### [Test Example 28: Body-Temperature-Increasing Function (After Taking Bath)]

Tested was body-temperature-increasing function in the case that the functional water of the present invention was used as a bath liquid (external preparation). Five healthy test subjects A to E (three males, two females, average age: 56 years old) were targeted. Test was conducted for 2 days in total. On day 1, each test subject measured the body temperature before taking a bath. Subsequently, each test subject took a bath at 41°C for 5 minutes, and thereafter measured the body temperature. On day 2, similarly, each test subject first measured the body temperature before taking a bath. Subsequently, 10 mL of the test solution of Example 1 (amount of polyfunctional amine: 0.75 mg) was added to a bath (41°C, volume of hot water: 200 L), and each test subject took a bath for 5 minutes, and thereafter measured the body temperature. Table 20 shows the results.

### [Table 20]

**(Table 20)**

| Body-temperature-increasing function (after taking bath) | | | | | | |
|---|---|---|---|---|---|---|
| Test subject | Sex | Age | Day 1 | | Day 2 | |
| | | | Body temperature before taking bath | Body temperature after taking common bath at 41°C for 5 minutes | Body temperature before taking bath | Body temperature after taking bath with addition of 10 mL of Example 1 at 41°C for 5 minutes |
| Test subject A | male | 71 | 36.1 | 36.6 | 36.2 | 37.1 |
| Test subject B | female | 67 | 35.8 | 36.2 | 35.9 | 36.9 |
| Test subject C | male | 44 | 36,3 | 36.8 | 36.3 | 37.2 |
| Test subject D | female | 33 | 36.1 | 36.5 | 36.0 | 37.0 |
| Test subject E | male | 65 | 36,2 | 36.7 | 36.1 | 37.1 |
| Average value | | | 36.1 | 36.56 | 36.1 | 37.06 |
| Average body temperature increase | | | | 0.46 | | 0.96 |

It was found that addition of the test solution of Example 1 as a bath liquid provides enhanced body-temperature-increasing effect corresponding to increase of approximately 0.5°C, as compared with a bath without addition of anything. It was demonstrated that use as a bath liquid (external preparation), as well as drinking, can provide comparable body-temperature-increasing function.

### [Test Example 29: Intraoral-Environment-Improving Function, Halitosis-Preventing Function]

Five healthy test subjects A to E (three males, two females, average age: 56 years old) were targeted, and allowed to self-check intraoral condition on the basis of the following evaluation criteria right after waking-up in the next morning of the following cases. Table 21 shows the results.
1. After tooth brushing before bedtime, the test solution of Example 1 was intraorally sprayed to such a degree that the inside of the mouth got wet.
2. After tooth brushing before bedtime, nothing was intraorally sprayed.

### [Evaluation Criteria]

∘: The inside of the mouth was not sticky at all.
Δ: The inside of the mouth was slightly or somewhat sticky.
×: The inside of the mouth was fairly or quite sticky.

### [Table 21]

**(Table 21)**

| Intraoral-environment-improving function | | | | |
|---|---|---|---|---|
| Test subject | Sex | Age | Without spraying | With spraying of Example 1 |
| Test subject A | male | 71 | Δ | ○ |
| Test subject B | female | 67 | Δ | ○ |
| Test subject C | male | 44 | × | ○ |
| Test subject D | female | 33 | Δ | ○ |
| Test subject E | male | 65 | × | ○ |

It was demonstrated that if the test solution of Example 1 is intraorally sprayed before bedtime, the inside of the mouse does not become sticky next morning, and a good intraoral environment is kept. It is inferred that because of this, halitosis at waking-up will be also suppressed.

### [Test Example 30: Antiinflammatory Function]

### Test subject (male, 70 years old)

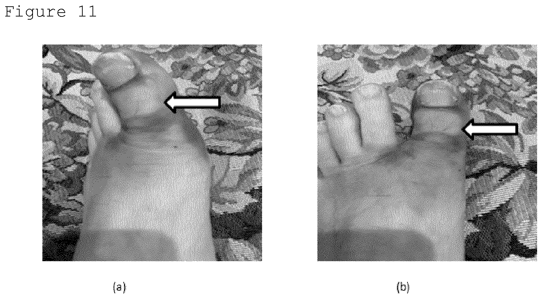

This test subject sprained his left big toe and felt significant pain. He searched for a cold patch but could not find it, and a commercially available adhesive plaster soaked in the test solution of Example 1 was attached in such a manner that the adhesive plaster would not dry. Next day, a phenomenon as shown in Figure 11 had occurred in the left big toe. When the adhesive plaster was removed from the part around the left big toe, where terrible internal bleeding had occurred, only the part with which the test solution of Example 1 had been in contact was completely free from internal bleeding. In an inference, the following two possibilities are contemplated and either one of them must be the reason: the possibility that contact with the test solution before the occurrence of internal breeding prevented the occurrence of internal bleeding; and the possibility that the bleeding occurred and simultaneously cured. Since internal bleeding occurs in tissue, it is appropriate to assume that internal bleeding is not affected by an externally attached object. Probably, the test solution of Example 1 inhibited bleeding at the affected part and exhibited the antiinflammatory function.

### [Test Example 31: Anticancer Function]

### Case 1:

### Test subject (female)

This test subject was under treatment of breast cancer by administration of an anticancer agent, and began to drink the test solution of Example 1 in an absolute dose of 30 mL/day (dose of polyfunctional amine: 2.25 mg/day). Before that, she had been staying in bed for about 3 days because of side effects, but the side effects became milder after drinking, and almost disappeared. On the next day of administration of an anticancer agent, she daringly traveled abroad, and returned home happily.

### Case 2:

### Test subject (female, 88 years old)

This test subject developed colorectal cancer at her high age of 88 and the colorectal cancer gradually progressed, although the progression was slow. Her son made her drink the test solution of Example 1 in an absolute dose of 30 mL/day (dose of polyfunctional amine: 2.25 mg/day), and after that the progression of the cancer almost stopped. At the time 3 years after the beginning of drinking, she is living happily.

### Case 3:

### Test subject (male)

An early-stage bladder cancer was found from this test subject, and his physician recommended resection to him. He began to drink the test solution of Example 1 in an absolute dose of 30 mL/day (dose of polyfunctional amine: 2.25 mg/day) together with ingestion of other health foods. After a month, he underwent endoscopic surgery to find no cancer, and since that he has been in such a cancer-free state.

### Case 4:

### Test subject (male)

A bladder cancer was found from this test subject, and he began to drink the test solution of Example 1 in an absolute dose of 30 mL/day (dose of polyfunctional amine: 2.25 mg/day). He had a tendency to easily get fatigued before that, but 3-month-drinking made his condition good and enabled him to work with less difficulty. As a result of drinking for approximately a year, the bladder cancer disappeared, and he is continuing drinking for prevention of metastasis (approximately 5 years).

### Case 5:

### Test subject (female)

The test subject was diagnosed with terminal lung cancer, and given a year to live. Three months after the diagnosis, she began to drink the test solution of Example 1 in an absolute dose of 30 mL/day (dose of polyfunctional amine: 2.25 mg/day). As a result of 3-month-drinking, her skin became clear and she recovered her energy. The progression of the cancer stopped, and her physical strength recovered in a year after the given life expectancy, and with gained confidence she traveled abroad for 10 days. Current diagnosis has revealed that the progression of the cancer has stopped and most part of the cancer has been killed.

### Case 6:

### Test subject (female)

This test subject was diagnosed with suspicious breast cancer through mammography in health checkup. She began to drink the test solution of Example 1 in an absolute dose of 30 mL/day (dose of polyfunctional amine: 2.25 mg/day). One month after the beginning of drinking, she underwent detailed examination, and diagnosis therein found nothing. She is continuing drinking for approximately 4 years.

### Case 7:

### Test subject (female)

This test subject, having progressing bladder cancer, began to drink the test solution of Example 1 in an absolute dose of 30 mL/day (dose of polyfunctional amine: 2.25 mg/day). In 3 months, the bladder cancer decreased in size. She is continuing drinking for approximately 4 years.

### Case 8:

### Test subject (female)

A breast cancer of 18 mm × 15 mm × 13 mm was found from this test subject. Treatment with an anticancer agent had no effect, and she began to suffer from side effects 4 months after the initiation of the treatment. For this reason, she began to drink the test solution of Example 1 in an absolute dose of 30 mL/day (dose of polyfunctional amine: 2.25 mg/day). In examination after approximately 2 weeks, her physician said to her that the cancer had shrunk to 1/3 to 1/4 of the original volume. After that, the physician told her that no surgery was needed, but she underwent surgery at her own request. The cancer resected consisted almost only of killed, dead cancer cells, and there was no live cancer cell.

### Case 9:

### Test subject (male)

This test subject was diagnosed with bladder cancer, and began to drink the test solution of Example 1 in an absolute dose of 30 mL/day (dose of polyfunctional amine: 2.25 mg/day). In diagnosis after 2 months, he was told that the progression had stopped, and increased the dose of drinking to 40 mL/day (dose of polyfunctional amine: 3.0 mg/day). Diagnosis 2 months thereafter revealed that the cancer had completely disappeared. He is continuing drinking for approximately 4 years.

### Case 10:

### Test subject (female)

A gastric cancer beyond cure was found from this test subject together with metastasis to the liver, and she was given 2 to 3 months to live in diagnosis. She was in such a state that only treatment with an anticancer agent was applicable and surgery was not applicable. The next month, she began to drink the test solution of Example 1 in an absolute dose of 30 mL/day (dose of polyfunctional amine: 2.25 mg/day) (30 mL of the test solution of Example 1 was diluted to 70 mL in taking), and after 2 months she was told in diagnosis with CT examination that the inoperable cancer in the stomach had decreased in size. Although side effects due to an anticancer agent were present, she was living fairly happily. In medical checkup after 4 months, she was told that the levels of all of the cancer markers had been lowered. She is continuing drinking for approximately 3 years.

### Case 11:

### Test subject (male)

This test subject was diagnosed with bladder cancer, and began to drink the test solution of Example 1 in an absolute dose of 30 mL/day (dose of polyfunctional amine: 2.25 mg/day). Examination after 4 months revealed that the cancer marker level had been significantly lowered and the cancer had almost disappeared. He is still continuing drinking to prevent the recurrence (approximately 3 years).

### Case 12:

### Test subject (female)

This test subject was diagnosed with lung cancer, and began to drink the test solution of Example 1 in an absolute dose of 30 mL/day (dose of polyfunctional amine: 2.25 mg/day). Detailed examination after 3 months revealed that the cancer had completely disappeared.

### Case 13:

### Test subject (male)

This test subject had inoperable terminal liver cancer derived from cirrhosis. Further, he was in such a state that metastasis of the cancer could metastasize to any part in the whole body with high probability through intrusion of the cancer into the blood, and began to drink the test solution of Example 1 in an absolute dose of 30 mL/day (dose of polyfunctional amine: 2.25 mg/day). Examination after approximately 3 months found that the progression had stopped and examination after approximately 4 months also found no progression, and administration of an anticancer agent was discontinued and the regimen was changed to that for hepatitis C. He is still living happily even after the given life expectancy. Recovery of physical strength was appreciated 10 months after the beginning of drinking, and hence he underwent surgery to extirpate three liver cancers.

### Case 14:

### Test subject (female)

This test subject underwent recurrence of lung cancer, and the cancer marker level increased to 6.8. She began to drink the test solution of Example 1 in an absolute dose of 30 mL/day (dose of polyfunctional amine: 2.25 mg/day). In 2 months, the cancer marker level declined to 4.9. In subsequent 3 months, the cancer marker level decreased to 4.4. In examination after 7 months, the cancer had completely disappeared.

### Case 15:

### Test subject (male, 70 years old)

This test subject was diagnosed with bladder cancer, and the kidney was extirpated. In approximately 1.5 years, about 10 cancers recurred in the lung through metastasis, and pleural effusion was additionally caused. One month after the recurrence, he began to drink the test solution of Example 1 in an absolute dose of 30 mL/day (dose of polyfunctional amine: 2.25 mg/day). In examination approximately 4 months after the beginning of drinking, the number of cancers that had metastasized decreased to three, and in addition the size of each cancer was unchanged from the original size. He was told by his physician that he may discontinue treatment with an anticancer agent, if desiring to do so. In diagnosis 6 months after the beginning of drinking, the pleural fluid that had accumulated in 80% of the left lung had been reduced, and the diagnosis determined that most of the residual cancers were dead. Presented was a perspective that the problem would be probably solved. At that time, the pleural fluid had completely disappeared, and he was diagnosed as being ameliorated.

### Case 16:

### Test subject (female, 60 years old)

This test subject was diagnosed with terminal pancreatic cancer, and given a year to live. Five months after the diagnosis, she began to drink the test solution of Example 1 in an absolute dose of 30 mL/day (dose of polyfunctional amine: 2.25 mg/day). In medical checkup 5 months after the beginning of drinking, she was told that the progression of the pancreatic cancer had stopped. Her body weight increased by 6 kg from that at the beginning of drinking, and she is living truly happily even after the life expectancy of a year. Her flatus was very malodorous before, but the smell has become almost nonodorous since she began to drink the present solution.

### Case 17:

### Test subject (male)

This test subject was diagnosed with malignant brain tumor, and he began to drink the test solution of Example 1 in an absolute dose of 30 mL/day (dose of polyfunctional amine: 2.25 mg/day). In 3 months, the malignant brain tumor completely disappeared.

### Case 18:

### Test subject (female, 50 years old)

This test subject was removed of a breast cancer through surgical extirpation 3 years ago. However, the cancer recurred and metastasized to the whole body including the bone, the liver, and the lung a year ago, and for this reason she began to drink the test solution of Example 1 in an absolute dose of 30 mL/day (dose of polyfunctional amine: 2.25 mg/day) in the last summer. Results of diagnosis after the lapse of a year from the beginning of drinking demonstrated amazing recovery, specifically, the number of residual cancers was two. Although it is needless to say that she is using an anticancer agent, a special injection for breast cancer, and so on in combination, she has now returned to her place of work.

### Case 19:

### Test subject (male, 65 years old)

This test subject developed prostate cancer 3 years ago, and he began to drink the test solution of Example 1 in an absolute dose of 30 mL/day (dose of polyfunctional amine: 2.25 mg/day) 2 years ago. His reduced body weight has increased. Although the PSA value has increased, he is becoming energetic without being treated with an anticancer agent.

### Case 20:

### Test subject (male, 75 years old)

This test subject was diagnosed with prostate cancer because of increase of the PSA value to 7. He was to be subjected to surgery at an appropriate opportunity. He began to drink the test solution of Example 1 in an absolute dose of 30 mL/day (dose of polyfunctional amine: 2.25 mg/day). In 3 months, the PSA value decreased to 0.8, and surgery was no longer needed. The diagnosis as having cancer was cancelled as well.

### Case 21:

### Test subject (male, 65 years old)

This test subject was diagnosed with liver cancer (stage IV). He began to drink the test solution of Example 1 in an absolute dose of 30 mL/day (dose of polyfunctional amine: 2.25 mg/day). At the time 4 months after the beginning of drinking, results of examination confirmed that the cancer had shrunk.

### Case 22:

### Test subject (male, 60s)

This test subject was diagnosed with rectal cancer (stage IVb). He began to drink the test solution of Example 1 in an absolute dose of 30 mL/day (dose of polyfunctional amine: 2.25 mg/day). In 3 months after the beginning of drinking, the CEA marker level increased nearly twice. The increase of the level was determined to be due to flow-out of the marker protein caused by disintegration of cancer cells. As a result of subsequent continuous drinking, the marker level had decreased on entering the fourth month, and the pain had been also mitigated.

### Case 23:

### Test subject (male, 60 years old)

This test subject was diagnosed with colorectal cancer (stage IV), and notified that surgery was not applicable because of metastasis found at four positions. Approximately a month after the diagnosis, treatment with an anticancer agent was initiated. Surgery was planned to be performed if the cancers that had metastasized disappeared therethrough. On the other hand, he began to drink the test solution of Example 1 in an absolute dose of 30 mL/day (dose of polyfunctional amine: 2.25 mg/day). Two months after the initiation of treatment with an anticancer agent (3 months after the beginning of drinking), results of examination showed that the cancers at the four positions had disappeared. His physician said to him that this was miraculous, and surgery was performed 3 months after the initiation of treatment with an anticancer agent. At that time, his body weight had increased by 10 kg, and his physical strength was maintained. The surgery revealed that a little cancer was left unremoved. Examination a month after the surgery found that the cancer left unremoved had eventually disappeared. He now stays in his normal state.

### Case 24:

### Test subject (female, 70s)

This test subject was diagnosed with pancreatic cancer at stage IV with the life expectancy being 6 months. Prior to treatment with an anticancer agent that was to be initiated the next month, she began to drink the test solution of Example 1 in an absolute dose of 30 mL/day (dose of polyfunctional amine: 2.25 mg/day). The treatment with an anticancer agent was completed with very little impact of side effects. CT conducted in May confirmed that the size of the cancer had decreased. She is continuing drinking for approximately a year.

### Case 25:

### Test subject (female, 65 years old)

A colorectal cancer of about 6 cm in size was found from this test subject, and metastasis to the lung and the uterus was simultaneously found. She was diagnosed as being beyond cure in a neighboring hospital, and transferred to a distant hospital handling medical treatment not covered by health insurance. At that time, she began to drink the test solution of Example 1 in an absolute dose of 30 mL/day (dose of polyfunctional amine: 2.25 mg/day). Despite that she had been told that surgery was difficult, she recovered to a state that allowed surgery. Resection of the colorectal cancer, extirpation of the uterus, and resection of a cancer of the diaphragm were performed. After that, she returned to her hometown, and went to a distant hospital once a week. Currently, she has no cancer, and no metastasis has been found.

### Case 26:

### Test subject (male, 72 years old)

Malignant polyps were found from the esophagus, large intestine, and stomach of this test subject. They were resected, but the cancer marker level was as high as **9.8,** and his physician suspected metastasis of cancer and continued treatment. He began to drink the test solution of Example 1 in an absolute dose of 30 mL/day (dose of polyfunctional amine: 2.25 mg/day). In 3 months after the beginning of drinking, the marker level decreased to 5.0. He is now under observation.

### Case 27:

### Test subject (male, 59 years old)

This subject had lung cancer, and was given half a year to live. Soon after the statement, he began to drink the test solution of Example 1 in an absolute dose of 30 mL/day (dose of polyfunctional amine: 2.25 mg/day). In 5 months after the beginning of drinking, the progression of the cancer stopped, and he is energetically going to work even after the lapse of 6 months. His appetite is growing to a troublesome degree, and he is even playing golf.

### Case 28:

### Test subject (female, 72 years old)

This test subject was diagnosed with lung cancer in medical checkup. Before taking an anticancer agent, she began to drink the test solution of Example 1 in an absolute dose of 30 mL/day (dose of polyfunctional amine: 2.25 mg/day). After 2 months, in order to take an anticancer agent, she underwent examination to find the disappearance of the cancer, and use of an anticancer agent was no longer needed.

### Case 29:

### Test subject (male, 70 years old)

This test subject was diagnosed with lung cancer at stage IV, and given half a year to live. Soon after the statement, he began to drink the test solution of Example 1 in an absolute dose of 30 mL/day (dose of polyfunctional amine: 2.25 mg/day). Results of medical checkup 8 months after the beginning of drinking showed that the progression of the cancer had stopped, and the size had slightly decreased. Now, he has recovered to such a degree that he can go to a driving range, thus regaining health.

### Case 30:

### Test subject (female, 70s)

This test subject was diagnosed with duodenal carcinoma, and it was determined for her to undergo surgery after 2 months. Immediately after being diagnosed with the cancer, she began to drink the test solution of Example 1 in an absolute dose of 30 mL/day (dose of polyfunctional amine: 2.25 mg/day). Two months after the beginning of drinking, she underwent surgery. Her physician, an authority on duodenal carcinoma in a private university hospital, sent the extirpated part to biopsy just in case, and the result revealed that the part was not cancer.

### Case 31:

### Test subject (female, 78 years old)

Ten years had passed since this test subject was affected by breast cancer. The focus was a circle of approximately 15 cm exposed on the surface of the breast, and was always bleeding. She began to drink the test solution of Example 1 in an absolute dose of 30 mL/day (dose of polyfunctional amine: 2.25 mg/day). In a month after the beginning of drinking, the lump became smaller. The weakened gum was increasingly strengthened since the beginning of drinking, and thus tooth extraction was no longer needed. While she had been suffering from abnormal condition of the ear, the condition of the ear also improved by spraying the test solution of Example 1 to the nose.

### Case 32:

### Test subject (female, 89 years old)

This test subject, a female having dementia, was affected by rectal cancer and given a life expectancy. On the basis of decision by her physician, she began to drink the test solution of Example 1 in an absolute dose of 30 mL/day (dose of polyfunctional amine: 2.25 mg/day), together with application of the test solution of Example 1 to the focus part. The dementia was ameliorated, and the cancer exposed from the rectum to the groin shrunk by the application.

### [Test Example 32: Antirheumatic Function]

### Case 1:

### Test subject (male, 59 years old)

When being a post working abroad, this test subject developed rheumatism, which caused difficulty even in getting up in the morning. He began to drink the test solution of Example 1 in an absolute dose of 50 mL/day (dose of polyfunctional amine: 3.75 mg/day), and the symptoms almost completely disappeared in approximately 3 months. Since 2 years passed after the beginning of drinking, he has been living without consulting a physician.

### Case 2:

### Test subject (female, 35 years old)

This test subject, who had rheumatism, was suffering from stiffening of her fingers lasting 1 hour after waking-up followed by fighting against pain all day. In such a situation, she began to drink the present solution (absolute dose of test solution of Example 1: 50 mL/day, dose of polyfunctional amine: 3.75 mg/day). The stiffness and pain disappeared and were absent since the next day of the beginning of drinking. After that, the present solution was used up and she failed to drink it, and the next day slight stiffness and pain recurred. She got scared and resumed drinking, and the stiffness and pain disappeared and were absent since the next day. Ever since, she is continuing drinking, and is not presenting any symptom.

### Case 3:

### Test subject (female, 70 years old)

This test subject had severe rheumatism, and was suffering from difficulty in walking and incapable of getting up in the morning. She began to drink the test solution of Example 1 in an absolute dose of 30 mL/day (dose of polyfunctional amine: 2.25 mg/day). Three-month-drinking made it easy for her to get up in the morning and relieved her of pain, and hence the daily dose of prescribed steroidal tablets was reduced from two tablets to half a tablet. Nevertheless, the symptoms are being mitigated. She has even become able to take a walk, and the severity has become "moderate" from "severe", further getting better to "mild".

### [Test Example 33: Anti-Parkinson Function]

### Test subject (male, 60 years old)

This test subject was diagnosed with Parkinson's disease, and he began to drink the test solution of Example 1 in an absolute dose of 30 mL/day (dose of polyfunctional amine: 2.25 mg/day). He was not able to allow his little finger to meet his thumb, but he became able to do so in 3 months after the beginning of drinking. He is still continuing drinking (approximately 8 months have passed), and feels getting better.

### [Test Example 34: Analgesic Function]

### Test subject (male, 65 years old)

This test subject was suffering from tremendous pain due to tooth caries, and his cheek was swollen. The test solution of Example 1 was sprayed to the affected part every 10 minutes, and the tooth pain ceased and the swelling also subsided in about an hour. Ever since, he has not consulted a physician.

### [Test Example 35: Antiasthmatic Function]

### Case 1:

### Test subject (female, 40s)

This test subject had a weak constitution and had been suffering from asthma for a long time. She began to drink the test solution of Example 1 in an absolute dose of 30 mL/day (dose of polyfunctional amine: 2.25 mg/day), and she became free from asthmatic attack in 2 days. Although swelling of the tonsil occurred, which was inferred to be a healing crisis, after that the swelling disappeared, and the asthma has also ceased.

### Case 2:

### Test subject (female, 60 years old)

This test subject was a female having difficulty in sweating with an asthmatic constitution, and she was suffering from incapability of sweating on the body and concentration of sweating on the face. She began to drink the test solution of Example 1 in an absolute dose of 30 mL/day (dose of polyfunctional amine: 2.25 mg/day), and she then became able to sweat on the body on day 4 to recover her normal body. In addition, the asthma ceased.

### [Test Example 36: Anti-Hair-Graying Function, Hair-Blackening Function]

### Test subject (male, 70 years old)

While this test subject was drinking the test solution of Example 1 in an absolute dose of 30 mL/day (dose of polyfunctional amine: 2.25 mg/day) for daily health promotion, his hair roots with gray hairs began blackening. Base parts of his hair became black, and thus his gray hair began to improve.

### [Test Example 37: Antigout Function]

### Test subject (male)

While being suffering from repeated gouty attacks, this test subject began to drink the test solution of Example 1 in an absolute dose of 30 mL/day (dose of polyfunctional amine: 2.25 mg/day). Ever since, he has experienced no attack for about 2 years, in spite that the uric acid level is such a value that an attack may occur at any time.

### [Test Example 38: Function Against Connective Tissue Disease]

### Test subject (male, 72 years old)

This test subject had connective tissue disease, and was long suffering from such symptoms that the fingertips got cold and the skin turned pale when it was cold. For this reason, he began to drink the test solution of Example 1 in an absolute dose of 30 mL/day (dose of polyfunctional amine: 2.25 mg/day). In approximately 2 months, his skin became free from turning pale.

### [Test Example 39: Anti-Alzheimer Function]

### Case 1:

### Test subject (male, 80 years old)

This test subject was a male having severe Alzheimer's disease, and almost incapable of talking. He began to drink the test solution of Example 1 in an absolute dose of 30 mL/day (dose of polyfunctional amine: 2.25 mg/day). After approximately 2 months, he became able to reply when his son called to him and to talk to a slight degree.

### Case 2:

### Test subject (female, 90s)

This test subject had dementia, and began to drink the test solution of Example 1 in an absolute dose of 30 mL/day (dose of polyfunctional amine: 2.25 mg/day). In approximately 3 months, her vacant eyes recovered vitality, and her facial expression became sharp. Her cognitive symptoms recovered, and the ability of conversation was gradually regained. The test solution of Example 1 was applied to a site affected by tinea pedis in her foot, and as a result the tinea pedis soon cured.

### [Test Example 40: Anti-Heart-Disease Function]

### Test animal subject (5-years-old dog)

This test animal subject had innate heart disease, and had lived with drugs for years. The dog was allowed to drink the test solution of Example 1 in an absolute dose of 30 mL/day (dose of polyfunctional amine: 2.25 mg/day). After approximately 6 months, when the dog was taken to a hospital, it was found that the heart disease had completely cured.

### [Test Example 41: Wound-Healing-Promoting Function]

A person underwent the onset of inflammation at the gingiva beneath an abutment tooth after implant treatment, and the inflammation did not cure in a year. To the affected part of the patient, the test solution of Example 1 was applied, and the patient was instructed to use it at home by spraying it to the affected part. As a result, the inflammation of the gingiva, which had developed an intractable wound, completely cured in a month. The test solution of Example 1 was applied to another patient with serious gingival inflammation in the same manner, and as a result the gingival inflammation cured in a month. The wound-healing-promotion function of the test solution of Example 1 was thus confirmed. These results are in reports by attending dentists.

### [Test Example 42: Blood-Glycose-Reducing Function, Blood-Pressure-Reducing Function, Ameliorating Function for Peripheral Nerve Disorder]

### Test subject (female, 70 years old)

This test subject was diagnosed with hypertension and diabetes mellitus, and began to drink the test solution of Example 1 (absolute dose: 20 mL/day, dose of polyfunctional amine: 1.5 mg/day). In examination 2 weeks after the beginning of drinking, the systolic blood pressure of approximately 150 mmHg had been reduced to around 135 mmHg. In addition, the blood glucose level had been reduced. While she used to regularly take an analgesic because of numbness and pain of her hands and legs, the numbness and pain of her hands and legs were ameliorated in 2 months, and thus she discontinued taking the analgesic. While she used to need a cane because of difficulty in walking due to the pain of her legs, she became able to walk without a cane. She is continuing drinking for approximately 6 months.

### [Test Example 43: Antidepressant Function, Antischizophrenic Function]

### Case 1:

### Test subject (male)

While this test subject, who had been affected by inertia due to depression, was continuing living home without going out, he began to drink the test solution of Example 1 in an absolute dose of 30 mL/day (dose of polyfunctional amine: 2.25 mg/day). In approximately 2 months, he recovered to able to live daily life cheerfully, and even to plan a new business by himself. In an inference, the inertia due to failure of neurotransmission caused for some reason was ameliorated by ingestion of the test solution of Example 1 to lead to the cheerfulness.

### Case 2:

### Test subject (female, 35 years old)

This test subject, who had been affected by depression, was going to a hospital regularly. She began to drink the test solution of Example 1 in an absolute dose of 30 mL/day (dose of polyfunctional amine: 2.25 mg/day). In a month after the beginning of drinking, she gradually became cheerful and able to give a smile.

### Case 3:

### Test subject (female, 28 years old)

This test subject, who had developed depression, was always gloomy and even not able to greet. She began to drink the test solution of Example 1 in an absolute dose of 30 mL/day (dose of polyfunctional amine: 2.25 mg/day). Everyone appreciated that she became cheerful in approximately 2 months after the beginning of drinking. In addition, she became able to greet properly.

### Case 4:

### Test subject (male, 30s)

This test subject was incapable of reading because of schizophrenia. He began to drink the test solution of Example 1 in an absolute dose of 30 mL/day (dose of polyfunctional amine: 2.25 mg/day). In approximately a month after the beginning of drinking, he got motivated to read and began to open a book for reading. His mother got great hope, and he is now continuing drinking.

### [Test Example 44: Symptom-Ameliorating Function for Other Diseases]

### Case 1:

### Test subject (male)

This test subject was affected by a disease presenting as gathering of mold in a part from the tongue to the throat, and the cause of the disease was that cells could not beat mold because of weakened resistance. He began to drink the test solution of Example 1 in an absolute dose of 30 mL/day (dose of polyfunctional amine: 2.25 mg/day). As a result of drinking for about a year, the disease completely cured. He also had leukoderma (an intractable disease presenting as progression of skin whitening) and skin interfaces were susceptible to erosion by ultraviolet rays, but he has become completely free from erosion, and the progression of whitening has also stopped.

### Case 2:

### Test subject (female, 28 years old)

This test subject had ankylosing spondylitis and fibromyalgia (orphan diseases that cause ossification of muscles), and there was no therapy effective for her. She began to drink the test solution of Example 1 (absolute dose of test solution of Example 1: 20 mL/day, dose of polyfunctional amine: 1.5 mg/day). In approximately 2 months after the beginning of drinking, the diseases completely cured.

### Case 3:

### Test subject (female, 60 years old)

This test subject was suffering from cryptogenic symptoms (unidentified complaint) including migraine that attacked once per 3 days, dropsy of her hands and legs, and low body temperature, which inhibited waking-up on some days. She began to drink the test solution of Example 1 in an absolute dose of 30 mL/day (dose of polyfunctional amine: 2.25 mg/day). In 3 months after the beginning of drinking, most of the symptoms disappeared, and she is now living happily.

### Case 4:

### Test subject (female, 25 years old)

Sinusitis affected this test subject and a potent drug was prescribed for her, but she did not get better. She thought that she could no longer continue use of such a potent drug, and began to use a weak drug. However, the symptoms worsened to worry her. When a nasal drop of the test solution of Example 1 was administered to her, pus flowed down to the mouth in several hours, and thereafter the symptoms improved. The symptoms did not worsen even with the weak drug, and good condition has been successfully kept.

### Case 5:

### Test subject (female, 69 years old)

This test subject was suffering from spinal canal stenosis, and eventually affected by difficulty in walking. At around that time, she began to drink the test solution of Example 1 in an absolute dose of 30 mL/day (dose of polyfunctional amine: 2.25 mg/day). In 2 months after the beginning of drinking, she recovered with complete disappearance of the symptoms.

### Case 6:

### Test subject (female, 36 years old)

Herpes labialis affected this test subject. The test solution of Example 1 was sprayed five times per day, and the herpes labialis cured in 3 days. In typical cases, it takes 7 to 8 days therefor with a drug prescribed by a physician.

### Case 7:

### Test subject (female, 25 years old)

Cataract affected this test subject 2 years ago, and was slowly progressed. She began to apply an eye drop of the test solution of Example 1 about three times per day. She used to be dazzled by headlights of oncoming cars, but now does not feel dazzling. She is feeling better in terms of subjective symptoms.

### Case 8:

### Test subject (female, 36 years old)

This test subject was bothered for 3 years by cryptogenic watery stools about 10 times per day, and she underwent examination of the large intestine; however, the cause was not revealed. She began to drink the test solution of Example 1 in an absolute dose of 30 mL/day (dose of polyfunctional amine: 2.25 mg/day), which resulted in loose stools twice per day.

### Case 9:

### Test subject (male, 39 years old)

This test subject was alternately repeating diarrhea and constipation for about 5 years, and he took an intestinal regulator prescribed by a hospital; however, the symptoms unchanged. He began to drink the test solution of Example 1 in an absolute dose of 30 mL/day (dose of polyfunctional amine: 2.25 mg/day), and the bowel movement became normal in 3 weeks, and the frequency of defecations changed to twice to three times per day.

### Case 10:

### Test subject animal (5-year-old dog)

Water accumulated in the left and right salivary glands of this test subject animal, and each salivary gland swelled to a size a little smaller than that of a marble. The dog was allowed to drink the test solution of Example 1 in an absolute dose of 30 mL/day (dose of polyfunctional amine: 2.25 mg/day), and in a month the swelling in the left side completely subsided and the swelling in the right side shrunk to some degree.

### Case 11:

### Test subject animal (3-year-old dog)

This test subject animal had weak skin with the abdomen skin being as if being sprayed with red powder, and an itch and eczema were caused when the abdomen skin got worse. The dog was allowed to drink the test solution of Example 1 in an absolute dose of 30 mL/day (dose of polyfunctional amine: 2.25 mg/day), which resulted in complete improvement.

### Case 12:

### Test subject (male, 32 years old)

The test solution of Example 1 was sprayed to a part presenting with atopic disease, and the skin became clear in about a week.

### Case 13:

### Test subjects (three females)

Through spraying the test solution of Example 1 to the face every morning and every evening, the skin was provided with smooth texture and became poreless. As a result, cosmetics other than powder have become unnecessary, and the skin is getting clearness.

### Case 14:

### Test subject (female, 59 years old)

Through spraying the test solution of Example 1 to her hair, the bases of her hair gradually became upright to give soft feeling.

### Case 15:

### Test subject (female, 59 years old)

Hyposphagma affected this test subject, and she applied an eye drop of the test solution of Example 1. Next morning, the hyposphagma had completely cured. Usually, it would take 2 weeks or more therefor with an eye drop from a hospital.

### Case 16:

### Test subject (male, 60 years old)

This test subject was diagnosed with amyotrophic lateral sclerosis (ALS). After 2 months, he began to drink the test solution of Example 1 in an absolute dose of 30 mL/day (dose of polyfunctional amine: 2.25 mg/day). In 4 months after the beginning of drinking, the progression almost stopped. He is to be observed for the transition.

### Case 17:

### Test subject (female, 70 years old)

This test subject was suffering from sudden hearing loss for years, and wearing a hearing aid on the ear for 5 years. She began to drink the test solution of Example 1 in an absolute dose of 30 mL/day (dose of polyfunctional amine: 2.25 mg/day). In a short time after the beginning of drinking, she became free from the onset of hearing loss, which allowed her to remove the hearing aid. In addition, her hair gradually became bouncing, and her bowel movement also got better.

### Case 18:

### Test subject (male, 72 years old)

This test subject was affected by hypertension and taking an antihypertensive. He began to drink the test solution of Example 1 in an absolute dose of 30 mL/day (dose of polyfunctional amine: 2.25 mg/day). The blood pressure began to reduce after the beginning of drinking, and he has now discontinued taking an antihypertensive. Numerical values relating to diabetes mellitus have fallen within normal values. The face has become free of sagging, and become resilient.

### Case 19:

### Test subject (female, 75 years old)

This test subject was lacking appetite, had difficulty in walking, tended to run a fever on moving, was muddled to such a degree that she could not see whether it was in the morning or in the evening, and tended to mumble with a weak voice in conversation. She began to drink the test solution of Example 1 in an absolute dose of 30 mL/day (dose of polyfunctional amine: 2.25 mg/day), and as a result she recovered her appetite and became able to eat up hospital meals, and the way of talking changed to a clear and crisp way as in her healthy days. She became free from running a fever even in rehabilitation and able to practice walking.

### Case 20:

### Test subject (female, 68 years old)

This test subject had Sjogren's disease, a disease that inhibits secretion of saliva to cause severe intraoral dryness, accepting the disease as a lifetime disease she had to struggle with. She began to drink the test solution of Example 1 in an absolute dose of 30 mL/day (dose of polyfunctional amine: 2.25 mg/day). Since then, she came to sometimes undergo gushing-out of saliva, which gave her hope.

### Case 21:

### Test subject (male, 40 years old)

This test subject had been in a state of "hikikomori" for 30 years since his elementary school ages. He could barely go to a convenience store for his part time job four times a week. He began to drink the test solution of Example 1 in an absolute dose of 30 mL/day (dose of polyfunctional amine: 2.25 mg/day). He became motivated to join an IT company in 2 months after the beginning of drinking, and then took the examination and passed it. He came to do the daily work over 8 hours or more per day, and even became sociable. About a year has passed after that, and now he is living a life as a working adult very well, without returning to the old him.

### Case 22:

### Test subject (male, 77 years old)

This test subject was affected by hearing loss as a late effect of cerebral infarction. He began to drink the test solution of Example 1 in an absolute dose of 30 mL/day (dose of polyfunctional amine: 2.25 mg/day). In about 2 months after the beginning of drinking, the hearing loss recovered.

### Case 23:

### Test subject (female, 50s)

Hyposphagma often affected this test subject, and it would take approximately 2 weeks therefor with an eye drop. In this opportunity, she applied an eye drop of the test solution of Example 1, and the next day the hyposphagma had cured.

### [Test Example 45: Health-Promoting Function]

### Test subject (male, 38 years old)

This test subject began to drink the test solution of Example 1 in an absolute dose of 30 mL/day (dose of polyfunctional amine: 2.25 mg/day). Before the beginning of drinking, he had an introverted character. In about 2 months after the beginning of drinking, however, he became able to live daily life very cheerfully, and came to be motivated to do the work. He is continuing drinking for approximately 6 months. The efficacy as a health-promoting agent is appreciated.

### [Test Example 46: Evaluation of Long-Term Safety]

### Test subject (male, 70 years old)

This test subject began to drink the test solution of Example 1 (absolute dose of Example 1: 30 mL/day, dose of polyfunctional amine: 2.25 mg/day) approximately 10 years ago. Continuous daily drinking did not cause any symptom like side effect, and many events good for health rather has come to occur, such as a phenomenon that excretion of toxins such as acetaldehyde readily becomes faster and loss of headache, his chronic illness. Accordingly, the drinking for a long period of time is determined to be not problematic.

### [Test Example 47: Life-Extending Function 4 for Cut Flowers (Carnation)]

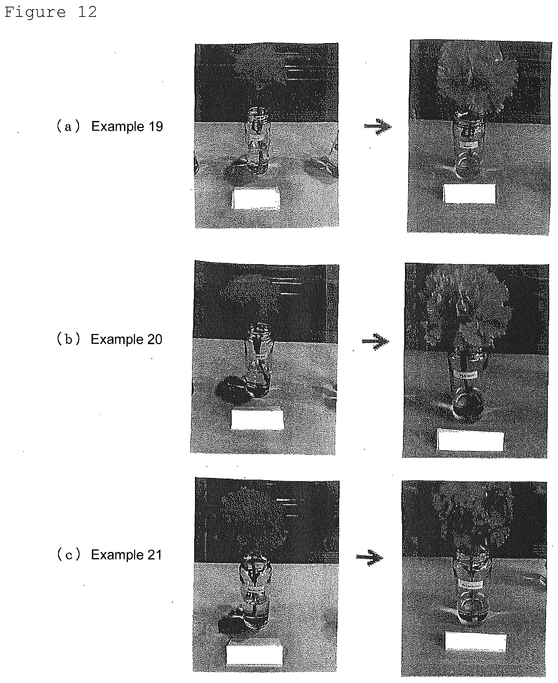

The test solutions of Examples 19 to 21 and Comparative Example 4 were prepared. Carnations were used as cut flowers, and each carnation was placed in a container in such a manner that the cut end was soaked in the test solution. The carnations were left to stand under such conditions that the carnations were irradiated with room light (fluorescent light) for approximately 10 hours in a day at a temperature of 20 ± 3°C. The state of each carnation 18 days after the start of the test was visually observed. During the test period, exchange of the test solutions was not performed. Figure 12 shows the results for Examples 19 to 21. (a): Example 19, (b): Example 20, and (c): Example 21. In each case, the left figure is a photograph at the start of the test, and the right figure is a photograph after 18 days.

The carnation with Comparative Example 4 began to bend down on day 10, and apparent color change began on day 14. No change was found for the carnations with Examples 19 and 20. For the carnation with Example 21, slight color change was found on day 18. From the results for Examples 19 to 21, the functional water of the present invention was revealed to have life-extending function for cut flowers of carnation.

### [Test Example 48: Antiseptic Function 2]

Four glass bottles respectively containing approximately 200 mL of the test solutions of Examples 19 to 21 and Comparative Example 4 were prepared, and an appropriate amount of beef was soaked in each test solution, and each glass bottle was covered with a lid and left to stand for 18 days. With Examples 19 to 21, the beef did not decompose at all. In the test with Comparative Example 4, decomposition began on day 5, and the beef completely decomposed on day 7.

### [Other Functions Expected]

The functional water of the present invention can be expected to synergistically exhibit various other functions (e.g., functions against other diseases, insect-bite-healing function, burn-healing function) through the above functions to contribute to health promotion. Since the functional water of the present invention has the above-described wide variety of physiological functions, the functional water of the present invention is said to be highly useful as a health-promoting agent.

Details of raw materials of the components used in Examples are as shown in Table 22.

### [Table 22]

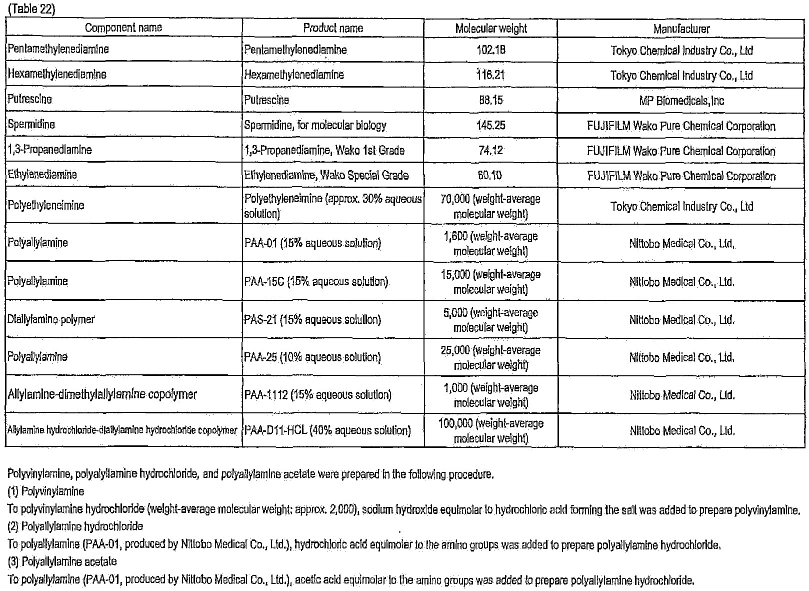

Variations of the present invention are additionally shown in the following.
[1] A freshness-keeping agent for foods, the freshness-keeping agent containing a polyfunctional amine and/or a salt thereof as a functional component, wherein the total content of water, the polyfunctional amine, and the salt of the polyfunctional amine is 95% by weight or more.
[2] An aging agent for foods, the aging agent containing a polyfunctional amine and/or a salt thereof as a functional component, wherein the total content of water, the polyfunctional amine, and the salt of the polyfunctional amine is 95% by weight or more.
[3] An antiseptic containing a polyfunctional amine and/or a salt thereof as a functional component, wherein the total content of water, the polyfunctional amine, and the salt of the polyfunctional amine is 95% by weight or more.
[4] A deodorant containing a polyfunctional amine and/or a salt thereof as a functional component, wherein the total content of water, the polyfunctional amine, and the salt of the polyfunctional amine is 95% by weight or more.
[5] A cleansing agent containing a polyfunctional amine and/or a salt thereof as a functional component, wherein the total content of water, the polyfunctional amine, and the salt of the polyfunctional amine is 95% by weight or more.
[6] A rust-preventing agent containing a polyfunctional amine and/or a salt thereof as a functional component, wherein the total content of water, the polyfunctional amine, and the salt of the polyfunctional amine is 95% by weight or more.
[7] A plant-growth-adjusting agent containing a polyfunctional amine and/or a salt thereof as a functional component, wherein the total content of water, the polyfunctional amine, and the salt of the polyfunctional amine is 95% by weight or more.
[8] A life-extending agent for cut flowers, the life-extending agent containing a polyfunctional amine and/or a salt thereof as a functional component, wherein the total content of water, the polyfunctional amine, and the salt of the polyfunctional amine is 95% by weight or more.
[9] A flowering-adjusting agent for cut flowers, the flowering-adjusting agent containing a polyfunctional amine and/or a salt thereof as a functional component, wherein the total content of water, the polyfunctional amine, and the salt of the polyfunctional amine is 95% by weight or more.
[10] A pest-controlling agent containing a polyfunctional amine and/or a salt thereof as a functional component, wherein the total content of water, the polyfunctional amine, and the salt of the polyfunctional amine is 95% by weight or more.
[11] A pest repellent containing a polyfunctional amine and/or a salt thereof as a functional component, wherein the total content of water, the polyfunctional amine, and the salt of the polyfunctional amine is 95% by weight or more.
[12] A parasite-preventing agent containing a polyfunctional amine and/or a salt thereof as a functional component, wherein the total content of water, the polyfunctional amine, and the salt of the polyfunctional amine is 95% by weight or more.
[13] An antimicrobial agent containing a polyfunctional amine and/or a salt thereof as a functional component, wherein the total content of water, the polyfunctional amine, and the salt of the polyfunctional amine is 95% by weight or more.
[14] An antiviral agent containing a polyfunctional amine and/or a salt thereof as a functional component, wherein the total content of water, the polyfunctional amine, and the salt of the polyfunctional amine is 95% by weight or more.
[15] A bowel-movement-improving agent containing a polyfunctional amine and/or a salt thereof as a functional component, wherein the total content of water, the polyfunctional amine, and the salt of the polyfunctional amine is 95% by weight or more.
[16] A fecal-odor-reducing agent containing a polyfunctional amine and/or a salt thereof as a functional component, wherein the total content of water, the polyfunctional amine, and the salt of the polyfunctional amine is 95% by weight or more.
[17] A blood-pressure-reducing agent containing a polyfunctional amine and/or a salt thereof as a functional component, wherein the total content of water, the polyfunctional amine, and the salt of the polyfunctional amine is 95% by weight or more.
[18] A body-temperature-increasing agent containing a polyfunctional amine and/or a salt thereof as a functional component, wherein the total content of water, the polyfunctional amine, and the salt of the polyfunctional amine is 95% by weight or more.
[19] A urinary-glucose-reducing agent containing a polyfunctional amine and/or a salt thereof as a functional component, wherein the total content of water, the polyfunctional amine, and the salt of the polyfunctional amine is 95% by weight or more.
[20] A blood-glycose-reducing agent containing a polyfunctional amine and/or a salt thereof as a functional component, wherein the total content of water, the polyfunctional amine, and the salt of the polyfunctional amine is 95% by weight or more.
[21] An anticancer agent containing a polyfunctional amine and/or a salt thereof as a functional component, wherein the total content of water, the polyfunctional amine, and the salt of the polyfunctional amine is 95% by weight or more.
[22] A side effect reducer for an anticancer agent, the side effect reducer containing a polyfunctional amine and/or a salt thereof as a functional component, wherein the total content of water, the polyfunctional amine, and the salt of the polyfunctional amine is 95% by weight or more.
[23] An antidepressant or antischizophrenic agent containing a polyfunctional amine and/or a salt thereof as a functional component, wherein the total content of water, the polyfunctional amine, and the salt of the polyfunctional amine is 95% by weight or more.
[24] An antiasthmatic agent containing a polyfunctional amine and/or a salt thereof as a functional component, wherein the total content of water, the polyfunctional amine, and the salt of the polyfunctional amine is 95% by weight or more.
[25] An antirheumatic agent containing a polyfunctional amine and/or a salt thereof as a functional component, wherein the total content of water, the polyfunctional amine, and the salt of the polyfunctional amine is 95% by weight or more.
[26] An anti-Parkinson agent containing a polyfunctional amine and/or a salt thereof as a functional component, wherein the total content of water, the polyfunctional amine, and the salt of the polyfunctional amine is 95% by weight or more.
[27] An antigout agent containing a polyfunctional amine and/or a salt thereof as a functional component, wherein the total content of water, the polyfunctional amine, and the salt of the polyfunctional amine is 95% by weight or more.
[28] An agent against connective tissue disease, the agent containing a polyfunctional amine and/or a salt thereof as a functional component, wherein the total content of water, the polyfunctional amine, and the salt of the polyfunctional amine is 95% by weight or more.
[29] An anti-Alzheimer agent containing a polyfunctional amine and/or a salt thereof as a functional component, wherein the total content of water, the polyfunctional amine, and the salt of the polyfunctional amine is 95% by weight or more.
[30] A hair-blackening-promoting agent containing a polyfunctional amine and/or a salt thereof as a functional component, wherein the total content of water, the polyfunctional amine, and the salt of the polyfunctional amine is 95% by weight or more.
[31] An anti-hair-graying agent containing a polyfunctional amine and/or a salt thereof as a functional component, wherein the total content of water, the polyfunctional amine, and the salt of the polyfunctional amine is 95% by weight or more.
[32] An intraoral-environment-improving agent or halitosis-preventing agent containing a polyfunctional amine and/or a salt thereof as a functional component, wherein the total content of water, the polyfunctional amine, and the salt of the polyfunctional amine is 95% by weight or more.
[33] An anti-heart-disease agent containing a polyfunctional amine and/or a salt thereof as a functional component, wherein the total content of water, the polyfunctional amine, and the salt of the polyfunctional amine is 95% by weight or more.
[34] A body-odor-preventing agent containing a polyfunctional amine and/or a salt thereof as a functional component, wherein the total content of water, the polyfunctional amine, and the salt of the polyfunctional amine is 95% by weight or more.
[35] An analgesic containing a polyfunctional amine and/or a salt thereof as a functional component, wherein the total content of water, the polyfunctional amine, and the salt of the polyfunctional amine is 95% by weight or more.
[36] A healing-promoting agent for insect bites, the healing-promotion agent containing a polyfunctional amine and/or a salt thereof as a functional component, wherein the total content of water, the polyfunctional amine, and the salt of the polyfunctional amine is 95% by weight or more.
[37] A burn-healing-promoting agent containing a polyfunctional amine and/or a salt thereof as a functional component, wherein the total content of water, the polyfunctional amine, and the salt of the polyfunctional amine is 95% by weight or more.
[38] An ameliorating agent for peripheral nerve disorder, the ameliorating agent containing a polyfunctional amine and/or a salt thereof as a functional component, wherein the total content of water, the polyfunctional amine, and the salt of the polyfunctional amine is 95% by weight or more.
[39] An antiinflammatory agent containing a polyfunctional amine and/or a salt thereof as a functional component, wherein the total content of water, the polyfunctional amine, and the salt of the polyfunctional amine is 95% by weight or more.
[40] The agent according to any one of [1] to [39], wherein the polyfunctional amine is at least one compound selected from the group consisting of a polyamine represented by formula (1): 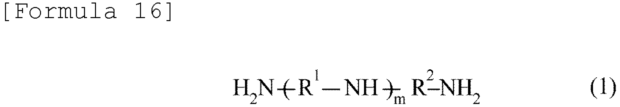
   wherein m represents an integer of 0 to 1000, R¹ and R² each independently represent a linear or branched alkylene group having two to eight carbon atoms, and if m is 2 or more, a plurality of R¹ are the same or different;
   a polymer having a structural unit derived from a cyclic amine represented by formula (2): 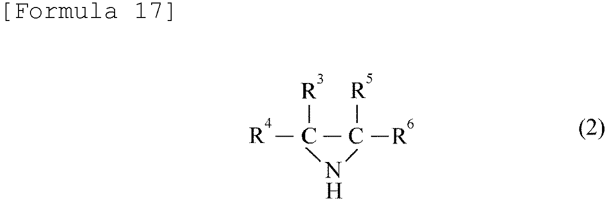
   wherein R³, R⁴, R⁵, and R⁶ each independently represent a hydrogen atom or a methyl group; and
   a polymer having a structural unit derived from an unsaturated amine represented by formula (3): 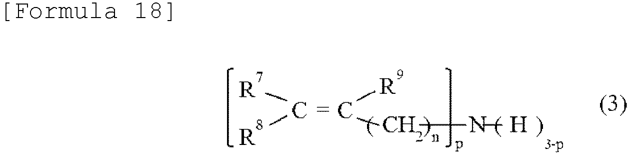
   wherein n represents an integer of 0 to 2, p represents an integer of 1 to 3, and R⁷, R⁸, and R⁹ each independently represent a hydrogen atom or a methyl group.
[41] The agent according to [40], wherein the total content of the polyfunctional amine and/or salt thereof is 0.001 to 9000 ppm by weight (e.g., 0.01 to 8000 ppm by weight, preferably 0.1 to 7000 ppm by weight, more preferably 0.5 to 6000 ppm by weight, especially preferably more than 0.8 ppm by weight (e.g., more than 1 ppm by weight, preferably more than 2 ppm by weight, more preferably more than 3 ppm by weight, even more preferably more than 4 ppm by weight, particularly preferably more than 5 ppm by weight) and not more than 5000 ppm by weight (e.g., 4000 ppm by weight or less, preferably 3000 ppm by weight or less, more preferably 2000 ppm by weight or less, even more preferably 1500 ppm by weight or less, particularly preferably 1000 ppm by weight or less)).
[42] The agent according to any one of [15] to [39], wherein the polyfunctional amine and/or salt thereof in a daily dose of 0.01 mg to 1500 mg (e.g., 0.1 mg to 1000 mg, preferably 0.2 mg to 500 mg, more preferably 0.5 mg to 100 mg) is ingested.
[43] A health-promoting agent containing a polyfunctional amine and/or a salt thereof as an active ingredient, wherein
   the polyfunctional amine is at least one compound selected from the group consisting of a polyamine represented by formula (1): 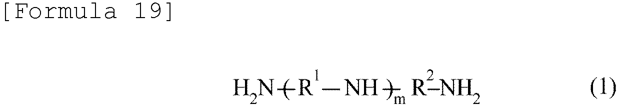
   herein m represents an integer of 0 to 1000, R¹ and R² each independently represent a linear or branched alkylene group having two to eight carbon atoms, and if m is 2 or more, a plurality of R¹ are the same or different;
   a polymer having a structural unit derived from a cyclic amine represented by formula (2): 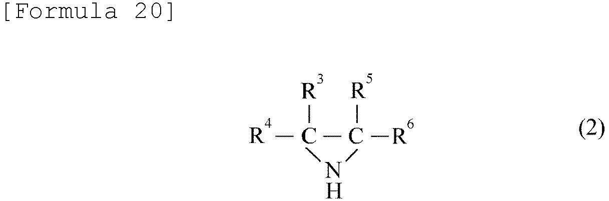
   wherein R³, R⁴, R⁵, and R⁶ each independently represent a hydrogen atom or a methyl group; and
   a polymer having a structural unit derived from an unsaturated amine represented by formula (3): 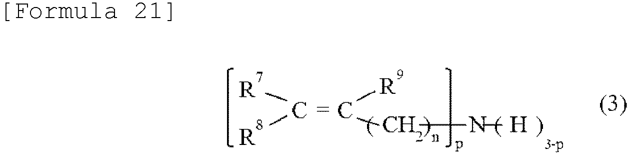
   wherein n represents an integer of 0 to 2, p represents an integer of 1 to 3, and R⁷, R⁸, and R⁹ each independently represent a hydrogen atom or a methyl group, and the total content of the polyfunctional amine and/or salt thereof is 0.001 to 9000 ppm by weight (e.g., 0.01 to 8000 ppm by weight, preferably 0.1 to 7000 ppm by weight, more preferably 0.5 to 6000 ppm by weight, especially preferably more than 0.8 ppm by weight (e.g., more than 1 ppm by weight, preferably more than 2 ppm by weight, more preferably more than 3 ppm by weight, even more preferably more than 4 ppm by weight, particularly preferably more than 5 ppm by weight) and not more than 5000 ppm by weight (e.g., 4000 ppm by weight or less, preferably 3000 ppm by weight or less, more preferably 2000 ppm by weight or less, even more preferably 1500 ppm by weight or less, particularly preferably 1000 ppm by weight or less)).
[44] The health-promoting agent according to [43], wherein the polyfunctional amine and/or salt thereof in a daily dose of 0.01 mg to 1500 mg (e.g., 0.1 mg to 1000 mg, preferably 0.2 mg to 500 mg, more preferably 0.5 mg to 100 mg) is ingested.

### Industrial Applicability

The functional water and health-promoting agent of the present invention can be prepared in a simple manner and exhibit excellent functions. The functional water and health-promoting agent of the present invention have a wide variety of functions applicable to many uses, and hence are highly useful for the industry, public health, and health of people.

## Claims

1. A functional water comprising a polyfunctional amine and/or a salt thereof as a functional component, wherein a total content of water, the polyfunctional amine, and the salt of the polyfunctional amine is 95% by weight or more.

2. The functional water according to claim 1, wherein the polyfunctional amine is at least one compound selected from the group consisting of a polyamine represented by formula (1): 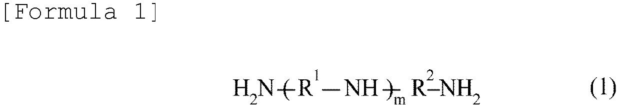
wherein m represents an integer of 0 to 1000, R¹ and R² each independently represent a linear or branched alkylene group having two to eight carbon atoms, and if m is 2 or more, a plurality of R¹ are the same or different;
a polymer having a structural unit derived from a cyclic amine represented by formula (2): 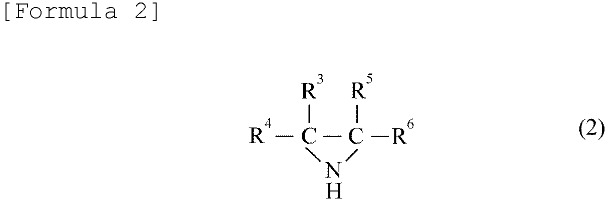
wherein R³, R⁴, R⁵, and R⁶ each independently represent a hydrogen atom or a methyl group; and
a polymer having a structural unit derived from an unsaturated amine represented by formula (3): 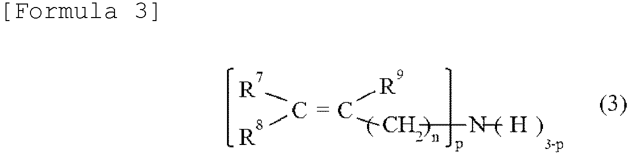
wherein n represents an integer of 0 to 2, p represents an integer of 1 to **3,** and R⁷, R⁸, and R⁹ each independently represent a hydrogen atom or a methyl group.

3. The functional water according to claim 1 or 2, wherein a total content of the polyfunctional amine and/or salt thereof is 0.0001 to 10000 ppm by weight.

4. The functional water according to any one of claims 1 to 3, having at least one function of freshness-keeping for foods, aging of foods, antisepsis, deodorization, cleansing, rust prevention, adjustment of plant growth, life extension for cut flowers, flowering adjustment for cut flowers, pest control, pest repellence, parasite prevention, antimicrobial function, antiviral function, improvement of bowel movement, reduction of fecal odor, reduction of blood pressure, increase of body temperature, reduction of urinary glucose, reduction of blood glucose, anticancer function, mitigation of side effects of anticancer agents, antidepressant function, antischizophrenic function, anti-heart-disease function, antiasthmatic function, antirheumatic function, anti-Parkinson function, antigout function, function against connective tissue disease, anti-Alzheimer function, promotion of hair blackening, anti-hair-graying function, improvement of an intraoral environment, prevention of halitosis, prevention of body odor, pain relief, promotion of healing of insect bites, promotion of wound healing, promotion of burn healing, amelioration of peripheral nerve disorder, and antiinflammatory function.

5. A material for preparing the functional water according to any one of claims 1 to 4, comprising a polyfunctional amine and/or a salt thereof.

6. The material according to claim 5, wherein a total content of the polyfunctional amine and/or salt thereof is more than 0.0001 ppm by weight and not more than 100% by weight based on a total amount of the material.

7. A health drinking water comprising a polyfunctional amine and/or a salt thereof as an active ingredient,
wherein
the polyfunctional amine is at least one compound selected from the group consisting of a polyamine represented by formula (1): 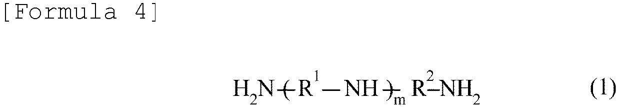
wherein m represents an integer of 0 to 1000, R¹ and R² each independently represent a linear or branched alkylene group having two to eight carbon atoms, and if m is 2 or more, a plurality of R¹ are the same or different;
a polymer having a structural unit derived from a cyclic amine represented by formula (2): 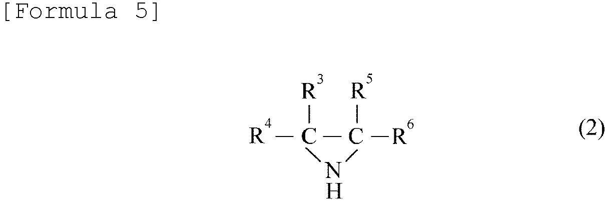
wherein R³, R⁴, R⁵, and R⁶ each independently represent a hydrogen atom or a methyl group; and
a polymer having a structural unit derived from an unsaturated amine represented by formula (3): 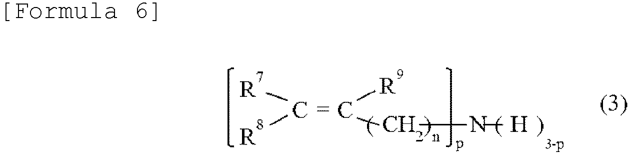
wherein n represents an integer of 0 to 2, p represents an integer of 1 to 3, and R⁷, R⁸, and R⁹ each independently represent a hydrogen atom or a methyl group.

8. The health drinking water according to claim 7, wherein a total content of the polyfunctional amine and/or salt thereof is 0.0001 to 10000 ppm by weight.

9. A bottled water, comprising the health drinking water according to claim 7 or 8 packed in a container.

10. A material for preparing the health drinking water according to claim 7 or 8, comprising a polyfunctional amine and/or a salt thereof.

11. The material according to claim 10, wherein a total content of the polyfunctional amine and/or salt thereof is more than 0.0001 ppm by weight and not more than 100% by weight based on a total amount of the material.

12. A health-promoting agent comprising a polyfunctional amine and/or a salt thereof as an active ingredient,
wherein
the polyfunctional amine is at least one compound selected from the group consisting of a polyamine represented by formula (1): 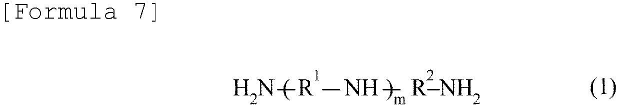
wherein m represents an integer of 0 to 1000, R¹ and R² each independently represent a linear or branched alkylene group having two to eight carbon atoms, and if m is 2 or more, a plurality of R¹ are the same or different;
a polymer having a structural unit derived from a cyclic amine represented by formula (2): 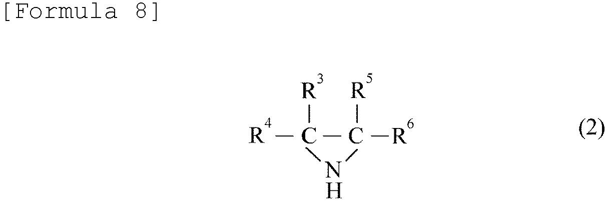
wherein R³, R⁴, R⁵, and R⁶ each independently represent a hydrogen atom or a methyl group; and
a polymer having a structural unit derived from an unsaturated amine represented by formula (3): 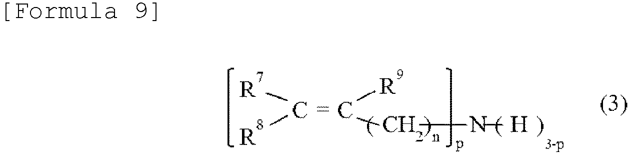
wherein n represents an integer of 0 to 2, p represents an integer of 1 to 3, and R⁷, R⁸, and R⁹ each independently represent a hydrogen atom or a methyl group.

13. The health-promoting agent according to claim 12, wherein a total content of the polyfunctional amine and/or salt thereof is 0.0001 to 10000 ppm by weight.
